(19) Europäisches Patentamt
European Patent Office
Office européen des brevets

(11) EP 3 020 361 B1

(12) **EUROPÄISCHE PATENTSCHRIFT**

(45) Veröffentlichungstag und Bekanntmachung des Hinweises auf die Patenterteilung:
**15.05.2019 Patentblatt 2019/20**

(51) Int Cl.:
***A61C 13/00*** *(2006.01)* ***A61K 6/083*** *(2006.01)*

(21) Anmeldenummer: **15193618.4**

(22) Anmeldetag: **09.11.2015**

(54) **VERWENDUNG RADIKALISCH HÄRTBARER ZUSAMMENSETZUNGEN IN GENERATIVEN FERTIGUNGSVERFAHREN**

USE OF RADICALLY HARDENING COMPOSITIONS IN GENERATIVE PRODUCTION METHODS

UTILISATION DE COMPOSITIONS A DURCISSEMENT RADICALAIRE DANS UN PROCEDE DE FABRICATION GENERATIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.11.2014 DE 102014116402**

(43) Veröffentlichungstag der Anmeldung:
**18.05.2016 Patentblatt 2016/20**

(73) Patentinhaber: **VOCO GmbH**
**27472 Cuxhaven (DE)**

(72) Erfinder:
- **Fontein, Nils, Dr.**
**27472 Cuxhaven (DE)**
- **Lübbe, Gerrit, Dr.**
**27476 Cuxhaven (DE)**
- **Oldenburger, Daniel**
**27476 Cuxhaven (DE)**
- **Plaumann, Manfred Thomas**
**27472 Cuxhaven (DE)**

(56) Entgegenhaltungen:
**DE-A1- 10 114 290** **DE-A1-102013 008 176**
**US-A1- 2003 069 326** **US-B1- 6 566 413**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 020 361 B1

Printed by Jouve, 75001 PARIS (FR)

## Beschreibung

Die vorliegende Erfindung betrifft die Verwendung radikalisch härtbarer Zusammensetzungen, umfassend mit radikalisch polymerisierbaren Gruppen substituierte kettenförmige und/oder zyklische und/oder in Käfigform vorliegende Polysiloxane mit mindestens 3 Siliziumatomen und/oder deren Mischformen, mit radikalisch polymerisierbaren Gruppen substituierte Disiloxane, optional ein, zwei, drei oder mehrere radikalisch härtbare Monomere ohne Siliziumatom, Füllstoffe, Initiatoren und/oder Katalysatoren für die radikalische Polymerisation sowie weitere übliche Additive, in generativen Fertigungsverfahren, vorzugsweise in der Stereolithographie (SL) und dem digital light processing (DLP). Die vorliegende Erfindung betrifft ferner die Verwendung der ausgehärteten radikalisch aushärtbaren Zusammensetzungen, vorzugsweise zur Herstellung von Dentalprodukten.

Weitere Aspekte der vorliegenden Erfindung und deren bevorzugte Ausgestaltungen ergeben sich aus der folgenden Beschreibung, den Ausführungsbeispielen und den Ansprüchen.

Der Begriff "generatives Fertigungsverfahren" ist ein feststehender Ausdruck (siehe hierzu A. Gebhard, "Generative Fertigungsverfahren" 4. Auflage, Carl Hanser Verlag, München 2013) und ist dem Fachmann auch als "Rapid Prototyping" (RP) bekannt. Er beschreibt, ausgehend von einem CAD-Datensatz als 3D-Geometriemodell, den schichtweisen, werkzeuglosen Aufbau eines jeweiligen Formkörpers.

Das heute am häufigsten verwendete RP Verfahren ist die Stereolithographie (siehe hierzu US 4,575,330). Diese Methode arbeitet mit einem ultravioletten Laser, der geeignete Harzsysteme schichtweise aushärtet. Im Allgemeinen werden klassische (Meth)acrylatharze, die mit geeigneten Photoinitiatoren versehen sind, polymerisiert. Eine vertikal bewegliche Plattform mit einer geeigneten Oberfläche (Metall, Glas, Keramik, etc.) zum Anwachsen der festen Harzphase wird auf den Abstand einer Schichtdicke (20 - 50 $\mu$m), gemessen vom Grund des flüssigen Harzbades, abgesenkt. Es erfolgt nun eine lokal punktuelle Vernetzung und Aushärtung des Harzes durch einen Laser, der beispielsweise von unten mithilfe von Mikrospiegeln ins Harzbad geführt wird. Das feste Polymerisat, das sich auf der Plattform bildet, wird nach der Vernetzung einer ersten Harzschicht um eine weitere Schichtdicke im Harzbad hochbewegt und somit erneut mit flüssigem Harz belegt. Der Laser härtet jetzt die zweite Schicht. Die Reihenfolge der Anhebung der mit Harzschichten bestückten beweglichen Plattform und das darauffolgende Polymerisieren werden so lange wiederholt, bis das gesamte 3D Modell aufgebaut ist.

Es ist charakteristisch für RP-Verfahren, dass die polymerisationsfähigen Gruppen während des schichtweisen Aufbaus der Formkörper nicht vollständig umgesetzt werden, so dass die resultierenden Teile noch keine ausreichende Endhärte und Festigkeit aufweisen. Die hergestellten Objekte müssen somit in einem weiteren Schritt einer thermischen Nachbehandlung im Ofen oder einer anschließenden Bestrahlung in einem Lichtkasten nachgehärtet werden.

DLP ist eine Modifizierung der SL. Anstelle eines Lasers wird bei diesem Verfahren sichtbares, nicht kohärentes Licht zur Polymerisation des härtbaren Harzes eingesetzt. Diese Technologie verwendet ein projiziertes Bild zur selektiven Polymerisation des Harzes. Das erforderliche Muster wird durch eine dynamische LCD Maske direkt auf das Harz abgebildet, so dass die gesamte Schicht gleichzeitig polymerisiert wird, wobei die belichteten Stellen der Schicht ausgehärtet werden und die unbelichteten Stellen unausgehärtet bleiben. Im Gegensatz zur Härtung durch den Laser, die nur punktuell erfolgt, ist dieses Verfahren somit deutlich schneller als SL.

Weitere RP-Verfahren sind die PolyJet-Technologie, die Galvanometer type scanning method, die Mikrostereolithographie, das Multi-Jet Modelling, selektives Lasersintern, 3D-Printing, Fused Deposition Modelling, 3D-Plotting, Laminated Object Manufacturing oder das Film Transfer Imaging.

Aufgrund der Vielseitigkeit der RP-Verfahren und des Vorteils dieser Verfahren im Vergleich zu abtragenden Fertigungsverfahren, beispielsweise in der effizienteren Herstellung sowie der nahezu unbegrenzten Geometriefreiheit der Bau- und Formteile, findet RP Einzug in ganze Industriebereiche wie Schmuck, Architektur, Design, Medizintechnik und hier insbesondere auch in der Zahnmedizin, wo es ja auf ganz korrekte Abformungs- und Duplizierverfahren ankommt.

So beginnt beispielsweise in der Zahnmedizin die Prozesskette mit der digitalen Erfassung der Mundsituation durch den 3D-Scan einer präparierten Kavität, um so auf Basis von CAD-Daten (Computer-Aided-Design) ein dentales Formteil zu fertigen. Das generierte Modell wird anschließend durch Verwendung eines formgebenden Verfahrens wie beispielsweise der Stereolithographie Schicht für Schicht aus einem flüssigen oder pastösen Harz hergestellt. Dentale Formkörper wie Inlays, Onlays, Veneers, Kronen, Brücken, künstliche Zähne, Zahnfertigteile, Gerüste, Provisorien sowie KFO-Produkte können direkt in der zahnärztlichen Praxis (oder im Dentallabor) ohne großen Aufwand oder Materialverlust (wie er bei der spanenden Bearbeitung von Rohlingen auftritt) angefertigt werden. Die Verfahren laufen einfach und schnell, wobei sie sogar gleichzeitig mehrere Form-/Bauteile aus verschiedenen Aufträgen fertigen können.

Generative Fertigungsverfahren, speziell auch stereolithographische Verfahren zur Herstellung dentaler Formkörper sind aus dem Stand der Technik bekannt.

In der DE 697 04 623 T2 wird ein Verfahren zur Herstellung eines dreidimensionalen Gegenstandes aus einem härtbaren flüssigen Medium angegeben, wobei der Gegenstand Schicht für Schicht aufgebaut wird, indem jedes Mal eine Schicht flüssigen Mediums auf einen Träger und/oder einem bereits geformten Teil des Gegenstandes in einem

flüssiges Medium enthaltenden Behälter angebracht wird und anschließend besagte Schicht gehärtet wird.

Die WO 2013/153183 A2 beschreibt Kompositharzzusammensetzungen und Verfahren zur Herstellung dentaler Bauteile mittels Stereolithographie. Beansprucht wird die Verwendung einer Dentalzusammensetzung, umfassend ein polyreaktives Bindemittel, zwei Photopolymerisationsinitiatoren mit unterschiedlichen Absorptionsmaxima und einen Absorber.

Die DE 199 38 463 A1 offenbart mit sichtbarem Licht aushärtbare Zusammensetzungen, enthaltend 2 - 99 Gew.-% eines härtbaren Harzes, 0,01 - 7 Gew.-% eines Initiators, 0 - 5 Gew.-% eines Koinitiators und 0 - 85 Gew.-% eines oder mehrerer Modifikatoren, wie Füllstoffen, Farbstoffen, Pigmenten, Fließverbesserern, Thixotropiemitteln, polymeren Verdickern, oxidierend wirkenden Zusatzstoffen, Stabilisatoren und Verzögerern zur Anwendung in einem formgebenden Verfahren. Genannt sind Verfahren der Mikroverfestigung, des RP, des Foliengießens, der Herstellung von Kunststoffsinterteilen, der Mikrostrukturierung, der Photolithographie, der Herstellung von Dentalprodukten, der Herstellung von chirugischen Implantaten und/oder der Herstellung von otoplastischen Produkten. In der Anmeldungsschrift ist der Aufbau einer stereolithographischen Apparatur beschrieben und abgebildet. In den erfindungsgemäßen Beispielen werden füllstofffreie radikalisch härtbare Zusammensetzungen auf Basis einer Mischung von 1,4-Butandioldimethacrylat, aliphatisches Diurethanmethacrylat und aliphatisches Urethanmethacrylat sowie einer Mischung von 1,4-Butandioldimethacrylat, aliphatisches Diurethanmethacrylat, aliphatisches Urethanmethacrylat und tetra-ethoxyliertes Bisphenol A Dimethacrylat eingesetzt.

Die DE 199 50 284 A1 ist größtenteils identisch zu der oben angegebenen DE 199 38 463 A1. Allerdings werden in dieser Anmeldungsschrift zusätzliche radikalisch härtbare Systeme aus den Druckschriften DE 41 33 494 C2 und DE 39 03 407 A1 auf Basis von Polysiloxanen in den Anmeldungstext integriert. Erfindungsgemäße Zusammensetzungen mit Polysiloxanen in Form von Beispielen enthält diese Anmeldungsschrift jedoch nicht.

Die DE 101 14 290 B4 zielt auf 3D-Plotting (ein alternatives Verfahren zur Stereolithographie) zur Herstellung von dentalen Formteilen, wobei härtbare Harze aus Düsen auf eine geeignete Bauplattform appliziert werden. In diesem Dokument werden als Harzsysteme auch SilikonHarze, die durch Kondensation oder Hydrolyse zu Polymernetzwerken führen, erwähnt.

Die DE 10 2012 012 346 A1 betrifft Formkörper aus weichbleibendem Dentalmaterial, insbesondere eine Zahnfleischmaske sowie Verfahren zu deren Herstellung mittels RP. Der Formkörper aus weichbleibendem Dentalmaterial, insbesondere die Zahnfleischmaske oder Unterfütterung für eine Dentalprothese, soll durch ein Licht härtendes RP-Verfahren einer Strahlen härtbaren Zusammensetzung, insbesondere mittels UV-A und/oder UV-B Strahlen, schichtweise zu einem Elastomer ausgehärtet werden können. Besonders bevorzugt sollen Zusammensetzungen umfassend Strahlen härtbare Silikone verwendet werden können, die weitere Hilfsstoffe, Füllstoffe, Pigmente oder Verdünner enthalten können. Hierbei können in einer Hydrosilylierungsreaktion mit mindestens zwei Alken-Gruppen funktionalisierte Silikone und hydridische Silikone mit mindestens zwei Si-H-Funktionalitäten in Gegenwart eines Hydrosilylierungskatalysator umgesetzt werden. Alternativ sollen auch silanolterminierte Polysiloxane und Silan-Vernetzungsmittel, ausgewählt aus Vinyltrimethoxysilan, Vinyltrioaminosilan, Vinyltriamidosilan, Vinyltrioximosilan, Vinyltriisopropenoxysilan oder Vinyltriacetoxysilan und einen Photoinitiator eingesetzt werden können.

Die in der DE 199 34 407 A1 angegebenen Silane sollen niedrig-viskos und flexibel sein, die alleine oder zusammen mit anderen hydrolysierbaren, kondensierbaren und/oder polymerisierbaren Komponenten zu Kieselsäurepolykondensaten oder zu Kieselsäureheteropolykondensaten verarbeitet werden können, deren endgültige Härtung dann durch Polymerisation der C=C Doppelbindungen erfolgen soll. Die erfindungsgemäßen, hydrolysier- und kondensierbaren Silane sollen in speziellen Anwendungen wie beispielsweise der Beschichtung von Substraten aus Metall, Kunststoff, Papier, Keramik (durch Tauchen, Gießen, Streichen, Spritzen, elektrostatisches Spritzen, Elektrotauchlackierung), zur Herstellung optischer, optoelektrischer und elektronischer Komponenten, zur Herstellung von Füllstoffen, zur Herstellung von kratzfesten, abriebfesten Korrosionsschutzbeschichtungen von Formkörpern, beispielsweise durch Spritzguß, Formgießen, Pressen, Rapid-Prototyping oder Extrusion oder zur Herstellung von Compositen, beispielsweise mit Fasern, Füllstoffen oder Geweben verwendet werden. Neben den Anwendungsgebieten der Optik, Elektronik, der Medizin, insbesondere der Dentalmedizin und der Optoelektronik findet auch der Bereich der Lebensmittelverpackung Erwähnung.

Bedingt durch die Verfahrensabläufe ermangelt es den verwendeten Harzsystemen aus dem Stand der Technik bei generativen Fertigungsverfahren, beispielsweise in der Stereolithographie, an der gewünschten Präzision in der geometrischen Ausgestaltung der Formkörper. Dies liegt daran, dass durch den Einsatz einer Strahlungsquelle bei der schichtweisen Polymerisation des Harzes durch gestreute und/oder abgelenkte Photonen auch Bereiche mitgehärtet werden, die außerhalb der vorgegebenen Form angesiedelt sind. Um die Dimensionsungenauigkeiten zu minimieren, werden in der Regel den radikalisch härtbaren Zusammensetzungen sogenannte "Absorber" zugesetzt. Dies sind Moleküle, die Strahlung absorbieren. In der Literatur sind diese Verbindungen auch als Stabilisatoren oder Inhibitoren bekannt. Verwendet werden in der Regel Benzotriazole, Triazine, Benzophenone. Weiterhin sind Salicylsäurederivate sowie die Hindered Amine Light Stabilizers (HALS) zu nennen. Auch anorganische Salze, wie nanoskaliges Titandioxid oder Zinkoxide können eine strahlenabsorbierende Funktion übernehmen. Durch ihren Einsatz kann die Formgenauigkeit

stark verbessert werden, allerdings werden durch diesen Zusatz auch die Durchhärtetiefe und die Umsetzung während der radikalischen Härtung verringert.

Es war daher Aufgabe der Erfindung ein Harzsystem zur Verfügung zu stellen, das eine deutlich höhere Formgenauigkeit in einem generativen Fertigungsverfahren, wie beispielsweise der Stereolithographie, aufweist. Zudem sollte das Harzsystem auch eine verbesserte Bioverträglichkeit im Vergleich zu herkömmlichen Bindemitteln zeigen. Darüber hinaus sollten die erfindungsgemäßen Harzsysteme eine sehr gute Benetzbarkeit auf unterschiedlichen Substraten, eine hohe Umsatzrate, eine geringe Wasseraufnahme, gute mechanische Festigkeit sowie einen äußerst geringen Schrumpf besitzen.

Erfindungsgemäß wird die gestellte Aufgabe gelöst durch die Verwendung radikalisch härtbarer Zusammensetzungen enthaltend

a.) mit radikalisch polymerisierbaren Gruppen substituierte kettenförmige und/oder zyklische und/oder in Käfigform vorliegende Polysiloxane mit mindestens 3 Siliziumatomen und/oder deren Mischformen, wobei die Polysiloxane a.) durch Hydrolyse oder Teilhydrolyse und anschließende Kondensation oder Cokondensation von ein, zwei, drei oder mehr Verbindungen $R^1_aR^2_bSiX_c$ mit
X: Halogen oder Alkoxy
$R^2$: Alkyl, Alkenyl, Aryl, Alkylaryl, Arylalkyl, wobei unterschiedliche $R^2$ gleich oder verschieden sein können,
$R^1$: YZ,
Z: radikalisch polymerisierbare Gruppe, ausgewählt aus den Strukturelementen $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ und $-NH-(C=O)-C(CH_3)=CH_2$,
wobei unterschiedliche Z gleich oder verschieden sein können,

a: 1 oder 2,
b: 0 oder 1,
c: 2 oder 3,

$$a + b + c = 4,$$

Y: ein Verbindungselement, das das Siliziumatom mit der radikalisch polymerisierbaren Gruppe verknüpft und aus einer Alkylengruppe besteht,
wobei die Alkylenguppe eine unsubstituierte, durchgängige, geradkettige oder verzweigte Kohlenwasserstoffkette ist oder
wobei die Alkylengruppe eine unsubstituierte, durch eine Urethangruppe, Harnstoffgruppe, Estergruppe, Thiourethangruppe oder Amidgruppe unterbrochene Kohlenwasserstoffgruppe ist oder
wobei die Alkylengruppe eine durch eine Hydroxylgruppe substituierte Kohlenwasserstoffgruppe ist oder diese Hydroxylgruppe verestert oder verethert ist oder wobei die Alkylengruppe eine durch ein Sauerstoffatom und/oder Stickstoffatom und/oder Schwefelatom und/oder Estergruppen und/oder Thioestergruppen unterbrochene und durch eine Hydroxylgruppe substituierte Kohlenwasserstoffgruppe ist oder diese Hydroxylgruppe verestert oder verethert ist und
wobei unterschiedliche Y gleich oder verschieden sein können und
wobei Y 20 oder weniger Kohlenstoffatome enthält und
wobei YZ so gewählt wird, dass Z immer möglichst viele Atome aufweist,
erhalten werden.

b.) mit radikalisch polymerisierbaren Gruppen substituierte Disiloxane der folgenden Struktur

$$R^1_aR^2_{(3-a)}Si-O-SiR^2_{(3-b)}R^1_b$$

mit

$R^2$: Alkyl, Alkenyl, Aryl, Alkylaryl, Arylalkyl, wobei unterschiedliche $R^2$ gleich oder verschieden sein können,
$R^1$: YZ,
Z: radikalisch polymerisierbare Gruppe, ausgewählt aus den Strukturelementen $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ und $-NH-(C=O)-C(CH_3)=CH_2$,
wobei unterschiedliche Z gleich oder verschieden sein können,

a: 1 oder 2,
b: 1 oder 2,

Y: ein Verbindungselement, das das Siliziumatom mit der radikalisch polymerisierbaren Gruppe verknüpft und aus einer Alkylengruppe besteht,
wobei die Alkylenguppe eine unsubstituierte, durchgängige, geradkettige oder verzweigte Kohlenwasserstoffkette ist oder
wobei die Alkylengruppe eine unsubstituierte, durch eine Urethangruppe, Harnstoffgruppe, Estergruppe, Thiourethangruppe oder Amidgruppe unterbrochene Kohlenwasserstoffgruppe ist oder
wobei die Alkylengruppe eine durch eine Hydroxylgruppe substituierte Kohlenwasserstoffgruppe ist und/oder diese Hydroxylgruppe verestert oder verethert ist oder
wobei die Alkylengruppe eine durch ein Sauerstoffatom und/oder Stickstoffatom und/oder Schwefelatom und/oder Estergruppen und/oder Thioestergruppen unterbrochene und durch eine Hydroxylgruppe substituierte Kohlenwasserstoffgruppe ist und/oder diese Hydroxylgruppe verestert oder verethert ist und
wobei unterschiedliche Y gleich oder verschieden sein können und
wobei Y 20 oder weniger Kohlenstoffatome enthält und
wobei YZ so gewählt wird, dass Z immer möglichst viele Atome aufweist,

c.) optional ein, zwei, drei oder mehrere radikalisch härtbare Monomere ohne Siliziumatom,

d.) 85 Gewichtsprozent oder weniger Füllstoffe bezogen auf das Gesamtgewicht der radikalisch härtbaren dentalen Zusammensetzung,

e.) Initiatoren und/oder Katalysatoren für die radikalische Polymerisation und

f.) weitere übliche Additive

in generativen Fertigungsverfahrenzur Herstellung eines dentalen Formteils, wobei das dentale Formteil ein Inlay, ein Onlay, ein Veneer, eine Krone, eine Brücke, ein künstlicher Zahn, ein Zahnfertigteil, ein Gerüst, ein Provisorium, eine Teil- oder Vollprothese sowie ein KFO-Produkt ist.

Bei der Verwendung einer bevorzugten erfindungsgemäßen Zusammensetzung in generativen Fertigungsverfahren handelt es sich um die Verwendung einer durch Licht induzierten radikalisch härtbaren Zusammensetzung.

Polysiloxanverbindungen mit mindestens 3 Siliziumatomen im Sinne des vorliegenden Textes besitzen mindestens eine oder mehrere Ketten, die alternierend angeordnete und miteinander verbundene Siliziumatome und Sauerstoffatome besitzen, wobei sich die Ketten auch zu Ringen unterschiedlicher Größe, bzw. zu noch umfangreicheren Strukturen wie Käfigen zusammenschließen und wobei an den Siliziumatomen organische Gruppen (organische Seitenketten) gebunden sind. Diese organischen Gruppen können chemisch sehr verschieden zusammengesetzt sein und dadurch zu einer Vielzahl von Polysiloxanverbindungen mit unterschiedlichen Eigenschaften führen. Häufig besitzen diese organischen Gruppen eine oder mehrere organisch polymerisierbare Gruppen (d.h. reaktive Gruppen), die sich mit z.B. einer oder mehreren organisch polymerisierbaren Gruppen einer anderen Polysiloxanverbindung umsetzen können und dadurch vernetzte polymerisierte Polysiloxanverbindungen bilden. Ketten-, Ring- und Käfigstrukturen können auch in Form gemischter Strukturen auftreten. Sie sind ebenfalls Bestandteil der erfindungsgemäßen dentalen Zusammensetzungen.

Polysiloxanverbindungen sind seit langem bekannt und z.B. erhältlich durch Hydrolyse und Kondensation von Silanen mit hydrolysierbaren Gruppen (siehe z.B. DE 27 58 414 A1) oder durch Hydrosilylierung von Allyl- oder Vinylverbindungen mit SiH-haltigen Verbindungen. Polysiloxanverbindungen können zu einer Vielzahl von Produkten weiterverarbeitet werden, wie z.B. Überzüge, Beschichtungen, Membranen oder Bulkmaterialien. Diese Weiterverarbeitung beruht dabei häufig auf einer Vernetzungsreaktion organisch polymerisierbarer Gruppen in den Polysiloxanverbindungen (z.B. (Meth)acrylatgruppen) und der daraus resultierenden Bildung vernetzter Polysiloxanverbindungen.

Unter "(Meth)acryl" ist im Rahmen des vorliegenden Textes sowohl "Acryl" als auch "Methacryl" zu verstehen.

Eine spezifische Gruppe von Polysiloxanverbindungen enthält in den organischen Gruppen (Seitenketten) neben einer organisch polymerisierbaren Gruppe zusätzliche freie polare funktionelle Gruppen wie z.B. Hydroxy- oder Carboxygruppen.

So betrifft DE 44 16 857 C1 hydrolysierbare und polymerisierbare Silane, Verfahren zu deren Herstellung sowie deren Verwendung zur Herstellung von Kieselsäure(hetero)polykondensaten und (Hetero)Polymerisaten. Hydrolysierbare, organisch modifizierte Silane finden eine breite Anwendung bei der Herstellung kratzfester Beschichtungen für die unterschiedlichsten Substrate, für die Herstellung von Füllstoffen, von Klebe- und Dichtungsmassen oder von Formkörpern. Diese Systeme sollen auch - so wie die Systeme aus der oben bereits zitierten DE 199 34 407 A1- für einen

RP-Einsatz geeignet sein.

Die DE 44 16 857 C1 offenbart den Einsatz von Kieselsäure(hetero)polykondensaten (Polysiloxanverbindungen) in härtbaren Dentalmaterialien. Die hier beschriebenen Polysiloxanverbindungen umfassen freie polare funktionelle Gruppen (z.B. Carboxy- oder Hydroxygruppen), die in der Lage sind, geeignete Metallionen / Übergangsmetallionen zu komplexieren (z.B. Ionen des Titans, Zirkoniums oder Zinns).

DE 198 60 364 C2 betrifft polymerisierbare Dentalmassen auf der Basis von zur Aushärtung befähigten Siloxanverbindungen, deren Verwendung und Herstellung. In dieser Druckschrift wird die Herstellung von cyclischen Polysiloxanen und deren Verwendung als Basis für polymerisierbare Massen beschrieben. Sie sollen trotz hoher Dichte an zur Polymerisation befähigten Gruppen eine niedrige Viskosität aufweisen, die eine hohe Füllstoffaufnahme ermöglicht, welche zu Massen mit geringem Polymerisationsschrumpf führt. Auch hier befinden sich in den organischen Seitenketten der beschriebenen Polysiloxane neben den polymerisierbaren Einheiten, freie polare Funktionen.

Die freien polaren funktionellen Gruppen, z.B. in den vorstehend genannten Polysiloxanverbindungen führen jedoch regelmäßig auch zu unerwünschten Eigenschaften. So hat sich gezeigt, dass die durch die (freien) polaren funktionellen Gruppen hervorgerufene Hydrophilie der Polysiloxanverbindungen zu einer erhöhten Wasseraufnahme in Gegenwart von Feuchtigkeit führt, wodurch die Nassfestigkeit des härtbaren Materials in nachteiliger Weise verringert wird. Vermutlich durch Aufbau interner Wasserstoffbrückenbindungen kommt es zu einer Erhöhung der Viskosität. Dies wirkt sich dann negativ auf die Handhabbarkeit bei der Herstellung der härtbaren Zusammensetzungen aus.

Es besteht ein beträchtliches Bedürfnis, Polysiloxanverbindungen weiter an die Anforderungen an ein modernes (härtbares bzw. gehärtetes) Material anzupassen und die vorstehend genannten Nachteile zu minimieren, bzw. auszuräumen. Derart angepasste Polysiloxanverbindungen sollen dabei verbesserte physikalische Eigenschaften besitzen, z.B. einen geringeren Polymerisationsschrumpf beim Polymerisieren/Vernetzen der Polysiloxanverbindungen (d.h. beim Aushärten), eine erhöhte Festigkeit und/oder eine begrenzte Wasseraufnahme bei gleichzeitig komfortabler Konsistenz und Viskosität des härtbaren Materials.

Erste Erfolge bei der Verbesserung der Polysiloxane konnten durch Addition bzw. Substitution unterschiedlicher Substrate an die freien polaren Funktionalitäten der oben beschrieben speziellen Polysiloxane erreicht werden.

EP 1 874 847 B1 betrifft ein Verfahren zur Herstellung von Silanen mit zwei, drei oder sogar mehr Struktureinheiten, die über eine urethan-, säureamid- und/oder carbonsäureestergruppenhaltige Brücke miteinander verknüpft sind und von denen jede mindestens einen organisch polymerisierbaren Rest und mindestens einen Silylrest enthält. Diese Silane sollen sich insbesondere zur Modifizierung der Eigenschaften von Kieselsäure(hetero)polykondensaten und silylgruppenhaltigen, organischen Polymeren eignen. Das offenbarte Verfahren soll sich auch zur Verbrückung bereits vorkondensierter Kieselsäure(hetero)polykondensate eignen.

Die in EP 1 874 847 B1 offenbarten Kieselsäure(hetero)polykondensate (Polysiloxanverbindungen) verfügen über eine freie Hydroxygruppe (d.h. eine freie polare funktionelle Gruppe). Diese freien Hydroxygruppen können mit einem Dicarbonsäurederivat oder Diisocyanat so reagieren, dass Hydroxylgruppen mit einem Dicarbonsäurederivat bzw. Diisocyanat eine Verknüpfung (Verbrückung) bilden. Solche verknüpften Polysiloxanverbindungen besitzen ein deutlich höheres Molekulargewicht, ohne dabei die Doppelbindungsdichte (bedingt durch die organisch polymerisierbaren (Meth)acrylatgruppen) wesentlich zu verringern. Unter Doppelbindungsdichte wird hier der Quotient aus der Anzahl der polymerisierbaren Doppelbindungen in einer Verbindung und dem Molekulargewicht dieser Verbindung verstanden. Das höhere Molekulargewicht wirkt sich positiv auf die Biokompatibilität und den Polymerisationsschrumpf beim Vernetzen der verknüpften Polysiloxanverbindungen aus. Gleichzeitig konnte die Hydrophobizität der Polysiloxanverbindungen gesteigert werden. Es konnte jedoch gezeigt werden, dass sich das höhere Molekulargewicht nachteilig auf die Viskosität der verknüpften Polysiloxanverbindungen auswirkt. Die Viskosität steigt mit dem Grad der Verknüpfung, d.h. mit dem Molekulargewicht deutlich an, so dass eine tolerierbare Verarbeitbarkeit bei der Herstellung eines entsprechenden härtbaren Materials, das solche verknüpften Polysiloxanverbindungen umfasst, schon bei recht geringem Verknüpfungsgrad nicht mehr zufriedenstellend gegeben ist.

EP 1 685 182 B1 betrifft Silane und daraus gebildete Kieselsäurepolykondensate und - teilkondensate, in denen ein an einem Silicium gebundener, organischer Rest vorhanden ist, der verzweigt ist und an jedem der beiden Zweige mindestens eine eigenständig organisch polymerisierbare Gruppe trägt oder an einem der beiden Zweige eine solche Gruppe, am anderen einen Rest mit einem weiteren Siliziumatom aufweist.

Auch die in EP 1 685 182 B1 offenbarten Polysiloxanverbindungen umfassen freie polare funktionelle Gruppen in Form von Hydroxygruppen. Durch Reaktion von Carbonsäure- oder Isocyanatderivate, die ihrerseits ebenfalls polymerisierbare Doppelbindungen umfassen (z.B. (Meth)acrylatgruppen), können so organisch polymerisierbare Gruppen an freie polare funktionelle Gruppen geknüpft werden. Diese Reaktionsprodukte besitzen regelmäßig eine erhöhte Festigkeit bei gleichzeitig gesteigerter Hydrophobizität und verbesserter Biokompatibilität durch das erhöhte Molekulargewicht.

Es konnte jedoch auch in diesen Fällen gezeigt werden, dass das Einführen zusätzlicher polymerisierbarer Doppelbindungen zu einem erhöhten Polymerisationsschrumpf beim Vernetzen der Polysiloxanverbindungen führt, da sich die Doppelbindungsdichte deutlich erhöht, die Erhöhung des Molekulargewichtes aber nur vergleichsweise gering ist.

WO 2013/041723 A1 offenbart hydrolysierbare und polymerisierbare Silane (einschließlich Kieselsäurepolykondensate, d.h. Siloxane) mit einstellbarer räumlicher Verteilung der funktionellen Gruppen sowie deren Verwendung. Die in WO 2013/041723 A1 offenbarte Lehre betrifft ein Verfahren zur Kettenverlängerung von über Kohlenstoff an Silizium gebundenen Resten in Silanen oder Siloxanen.

WO 2013/053693 A1 offenbart Kieselsäurepolykondensate (Siloxane) mit cyclischen olefinhaltigen Strukturen und Verfahren zu deren Herstellung sowie deren Verwendung. WO 2013/053693 A1 offenbart, dass sich Polymerwerkstoffe mit in weiten Grenzen einstellbaren E-Moduln bei hoher elastischer Dehnung (d.h. ohne Sprödverhalten) und damit einer hohen Bruchzähigkeit aus Kieselsäure(hetero)polykondensaten mit cyclischen olefinhaltigen Strukturen herstellen lassen.

In der noch nicht offengelegten DE 10 2014 210 432 sind Polysiloxanverbindungen beschrieben, die die vorstehend genannten Nachteile aus dem Stand der Technik in einer härtbaren bzw. gehärteten Zusammensetzung nicht oder zumindest nur noch abgeschwächt aufweisen. Der konzeptuelle Ansatz dieser Systeme sieht vor, die freie funktionelle Gruppe im Silan so umzusetzen, dass keine zusätzlich polymerisierbaren Doppelbindungen ins System eingeführt werden. Stattdessen werden Kohlenwasserstoffreste hohen Molekulargewichts mit wenigstens 11 C-Atomen in das System eingebaut. Überraschenderweise zeigten sich bei diesen härtbaren Zusammensetzungen

- eine gute Viskosität der Polysiloxanverbindungen (die Viskosität sollte 50 Pa*s oder weniger bei einer Temperatur von 25°C betragen) und eine damit einhergehende hervorragende Verarbeitbarkeit bei der Herstellung eines die Polysiloxanverbindungen enthaltenden härtbaren Materials,
- eine gute Hydrophobizität,
- eine gute Festigkeit, insbesondere eine gute Biegefestigkeit,
- einen sehr geringen Polymerisationsschrumpf beim Vernetzen der Polysiloxanverbindungen, d.h. beim Aushärten des härtbaren Materials und
- eine gute Biokompatibilität.

Durch die in der DE 10 2014 210 432 ergriffenen Maßnahmen wurden somit gleich mehrere Probleme gelöst:

- durch Eliminierung polarer funktioneller Gruppen wurde die Ausbildung intermolekularer Wechselwirkungen unterbunden. Es gelang somit, die Viskosität des Systems trotz eines beachtlichen Molekuklargewichtszuwachses auf einem vergleichsweise niedrigen Niveau zu halten.
- durch Einbau von Kohlenwasserstoffresten relativ hohen Molekulargewichts wurde das Polysiloxanstrukturgerüst räumlich intramolekular aufgeweitet, so dass die Zugänglichkeit der radikalisch polymerisierbaren Gruppen während der Härtung erhöht wurde und damit die Umsetzungsrate optimiert werden konnte. Wie sonst könnte man den Umstand erklären, dass in diesen Systemen bei vergleichsweise reduzierter Doppelbindungsdichte die Festigkeit der Materialien, beispielsweise die Biegefestigkeit der gehärteten dentalen Zusammensetzungen auf einem sehr guten Niveau verbleiben und in vielen Fällen sogar erhöht sind im Vergleich zu den nicht weiter umgesetzten Polysiloxanen.
- durch die Molekulargewichtserhöhung bei Belassen der Funktionalität, also bei einer effektiven Absenkung der Doppelbindungsdichte konnte insbesondere die vielleicht wichtigste technische Kenngröße einer härtbaren Zusammensetzung, nämlich der Wert des Volumenschrumpfs während der Aushärtung, auf einen extrem niedrigen Wert eingestellt werden.
- Durch Einbau von Kohlenwasserstoffresten relativ hohen Molekulargewichts wurde das Polysiloxanstrukturgerüst auch vergleichsweise hydrophober gemacht, so dass die nicht erwünschte Wasseraufnahme nunmehr extrem geringe Werte annimmt.

In eigenen Untersuchungen wurde nun festgestellt, dass sich radikalisch härtbare Zusammensetzungen, die Polysiloxane enthalten, und zwar sowohl die neuen Polysiloxane aus der DE 10 2014 210 432 als auch die zuvor beschriebenen Systeme aus dem älteren Stand der Technik, weiter enorm verbessern lassen, wenn man ihnen radikalisch polymerisierbare Disiloxane zusetzt. Überraschenderweise wurde jetzt gefunden, dass durch die Anwendung solcher Zusammensetzungen in generativen Fertigungsverfahren, wie beispielsweise der Stereolithographie, die Genauigkeit im Aufbau der Formkörper ganz enorm verbessert werden kann.

Der Einsatz von Disiloxanen in radikalisch härtbaren Zusammensetzungen, deren chemischer Aufbau auf den klassischen vernetzenden "Monomeren" wie Bis-GMA (2,2 Bis[p-(2'-hydroxy-3'-methacryloxypropoxy)-phenyl]propan), UDMA (1,6-Bis(methacryloxy-2-ethoxycarbonylamino)-2,4,4-trimethylhexan), TEGDMA (Triethylenglykoldimethacrylat), HEDMA (Hexandioldimethacrylat), etc. beruht, ist aus dem Stand der Technik bekannt.

In einer Publikation mit dem Titel "Synthesis and properties of a polyfluorinated prepolymer multifunctional urethan methacrylate" (J.M. Antonuci, J.W. Stansbury, S. Venz, "Polymeric Materials Science and Engineering", 59, 388-396 (1988)), in der sogenannte "low surface energy resins" untersucht werden, wird das Bis-(methacryloxypropyl)tetramethyldisiloxan (BIS-MPTMS) zusammen mit Triethylenglykoldimethacrylat (TEGDMA) und 1,6-Hexamethylendimethacrylat (HEDMA) als organische Harzmatrix in einer Kompositzusammensetzung in Form eines Pulver/Flüssigkeitssystems verwendet. Auf der Seite 390 findet sich im Abschnitt "Formulation of composites" der Hinweis, dass BIS-MPTMS ein ausgezeichnetes Mittel ist, die Viskosität zu verringern und eine voluminöse, flexible Struktur aufweist. Es sei über einen großen Konzentrationsbereich mit vielen Harzen mischbar.

In einem Abstract mit dem Titel "Evaluation of siloxane containing dental composites" (J.S. Kuo, J.M. Antonucci, W. Wu, "Journal of Dental Research Abstracts", 6A, and Abstract No.30 (1985)) wird das BIS-MPTMS gemeinsam mit Bis-GMA und UDMA als organische Harzmatrix verwendet. Auch hier findet sich der Hinweis, BIS-MPTMS sei mit den üblichen Basismonomeren über einen großen Bereich (10 - 50 Gew.-%) mischbar. Das Fazit dieser Untersuchung lautet: "Mechanical properties of the siloxane-containing composites were almost comparable to the controls, but, significantly, had reduced WS (water sorption) and enhanced OER (oral environmental resistance)."

In einer neueren Veröffentlichung mit dem Titel "Synthesis of none Bisphenol A structure dimethacrylate monomer and characterization for dental composite applications" (X. Liang, F. Liu, J. He, "Dental Materials", 30, 917 - 925, (2014)) wurde das Reaktionsprodukt (SiMA) der ringöffnenden Additionsreaktion zwischen 1,3-Bis[2(3,4-epoxycyclohex-1-yl)ethyl]tetramethyldisiloxan und Methacrylsäure als Bis-GMA-Alternative zusammen mit TEGDMA als organische Matrix in dentalen Kompositen untersucht. Die Autoren schlussfolgern, dass die "study of SiMA based resin and composite material showed that SiMA had potential to be used in clinic, but mechanical properties of SiMA based resin and composite needed to be improved..."(Seite 923, Punkt 5, "conclusion").

Keines dieser Dokumente legt die Verwendung von radikalisch härtbaren Zusammensetzungen umfassend die Kombination von mit radikalisch polymerisierbaren Gruppen substituierten kettenförmigen und/oder zyklischen und/oder in Käfigform vorliegenden Polysiloxanen mit mindestens 3 Siliziumatomen und/oder deren Mischformen mit radikalisch polymerisierbaren Gruppen substituierten Disiloxanen der oben bezeichneten Struktur in generativen Fertigungsverfahren nahe.

<u>Bestandteil a.)</u> - mit radikalisch polymerisierbaren Gruppen substituierte kettenförmige und/oder zyklische und/oder in Käfigform vorliegende Polysiloxane mit mindestens 3 Siliziumatomen und/oder deren Mischformen.

Mit radikalisch polymerisierbaren Gruppen substituierte kettenförmige und/oder zyklische und/oder in Käfigform vorliegende Polysiloxane mit mindestens 3 Siliziumatomen und/oder deren Mischformen können über den Sol-Gel Prozeß durch gezielte Hydrolyse und Kondensation von entsprechend funktionalisierten Derivaten von Alkoxiden des Siliziums oder von Halogensilanen synthetisiert werden. Diese Herstellverfahren sind in der Literatur vielfach beschrieben. In der Regel geht man bei einer solchen Synthese von einem Standardsilan wie beispielsweise dem Isocyanatopropyldiethoxysilan aus, das in einem ersten Schritt, ebenfalls in einer Standardreaktion, beispielsweise in einer Isocyanat-Alkohol Polyaddition, beispielsweise mit dem Glycerin-1,3-dimethacrylat zum entsprechenden Urethan umgesetzt wird. Die hierbei erhaltene Verbindung besteht auf der einen Seite aus dem Siliziumatom, das mit hydrolysier- und kondensierbaren Gruppen bestückt sind und das über einen sogenannten Abstandshalter, bestehend aus einer Alkylgruppe (hier eine Propylgruppe) und einer Urethangruppe als strukturelles Verbindungselement zu einem weiteren funktionellen Struktursegment, in diesem Fall zu zwei radikalisch polymerisierbaren Methacrylatgruppen übergeht. Ein solch einfaches Syntheseverfahren kann in vielfacher Weise modifiziert werden, da die Reaktionsmöglichkeiten zwischen entsprechend funktionalisierten Silanen und geeigneten Reaktanden unbegrenzt erscheinen. Entsprechend groß sind die Synthesevorschläge in der Literatur. Die Ausgangsverbindung umfasst somit ein anorganisch kondensierbares Strukturelement, ein variabel gestaltbares Verbindungselement sowie ein radikalisch vernetzbares organisches Grundgerüst. In einer katalytisch gesteuerten Hydrolyse und Kondensation wird das Polysiloxan als ein anorganisches Kondensat, substituiert mit radikalisch polymerisierbaren Gruppen, gewonnen. Ob das Polykondensat in Form von Ketten, Ringen oder dreidimensionalen Käfigformen, bzw. in den entsprechenden Mischformen vorliegt, hängt von den genauen Bedingungen der Kondensation ab. Dazu zählen neben den Reaktionsbedingungen (pH-Wert, Menge an Lösungsmittel und Wasser, Art und Menge des Katalysators, Reaktionstemperatur, Art der Aufbereitung, etc.) auch die Strukturformen des Ausgangssilans, wobei die Anzahl der Alkoxygruppen, die Anzahl der radikalisch polymerisierbaren Gruppen, die chemische Art des Verbindungselements sowie die Kettenlänge des Abstandshalters von Bedeutung sind. Angaben hierzu finden sich sowohl in der wissenschaftlichen Literatur wie in der Patentliteratur.

Die Polysiloxane verfügen als Bindeglied zwischen anorganischer und organischer Chemie über besondere Materialeigenschaften. Da sie zudem physiologisch inert sind, also keine nennenswerte Toxizität besitzen, sind sie speziell für Anwendungen in der Medizin bedeutsam.

Grundlage für die kaum vorhandene Toxizität der Polysiloxane ist die geringe biologische Angreifbarkeit der Silizium-Kohlenstoffbindungen und die eingeschränkte Diffusionsfähigkeit der stark hydrophoben Polymerketten durch Zellmembranen, weshalb sie sich besonders zur Implantation (in Zähne) eignen sollten.

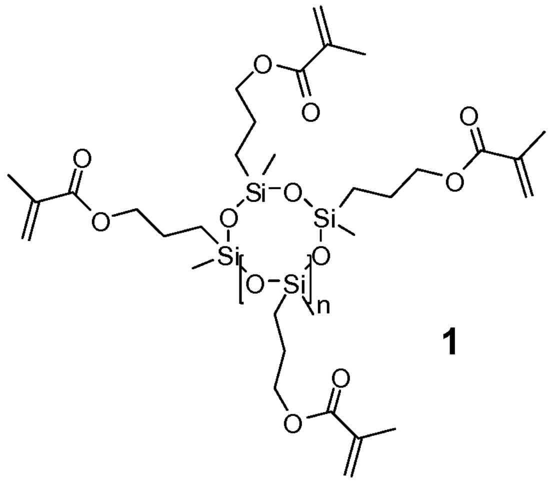

1

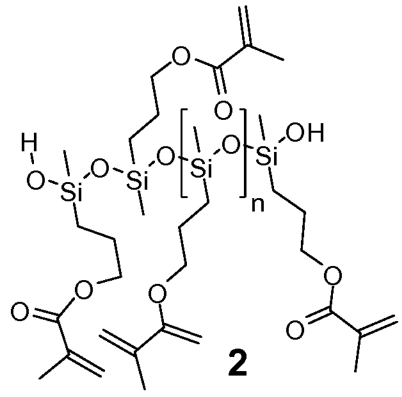

2

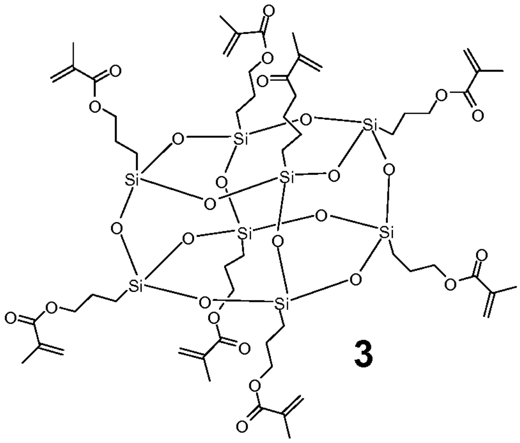

3

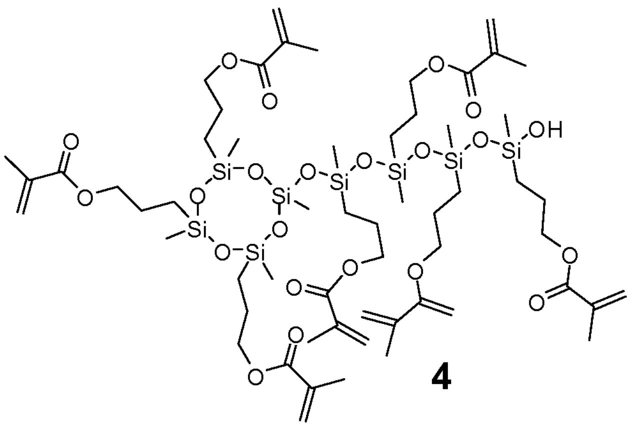

4

Bestandteil b.) - mit radikalisch polymerisierbaren Gruppen substituierte Disiloxane der folgenden Struktur $R^1_aR^2_{(3-a)}Si\text{-}O\text{-}SiR^2_{(3-b)}R^1_b$ mit
$R^2$: Alkyl, Alkenyl, Aryl, Alkylaryl, Arylalkyl, wobei unterschiedliche $R^2$ gleich oder verschieden sein können,
$R^1$: YZ,
Z: radikalisch polymerisierbare Gruppe, ausgewählt aus den Strukturelementen $-O-(C{=}O)-CH{=}CH_2$, $-O-(C{=}O)-C(CH_3){=}CH_2$, $-(C{=}O)-CH{=}CH_2$, $-(C{=}O)-C(CH_3){=}CH_2$, $-CH{=}CH_2$, $-C(CH_3){=}CH_2$, $-NH-(C{=}O)-CH{=}CH_2$ und $-NH-(C{=}O)-C(CH_3){=}CH_2$,
wobei unterschiedliche Z gleich oder verschieden sein können,

- a: 1 oder 2,
- b: 1 oder 2,

Y: ein Verbindungselement, das das Siliziumatom mit der radikalisch polymerisierbaren Gruppe verknüpft und aus einer Alkylengruppe besteht,
wobei die Alkylenguppe eine unsubstituierte, durchgängige, geradkettige oder verzweigte Kohlenwasserstoffkette ist oder
wobei die Alkylengruppe eine unsubstituierte, durch eine Urethangruppe, Harnstoffgruppe, Estergruppe, Thiourethangruppe oder Amidgruppe unterbrochene Kohlenwasserstoffgruppe ist oder
wobei die Alkylengruppe eine durch eine Hydroxylgruppe substituierte Kohlenwasserstoffgruppe ist und/oder diese Hydroxylgruppe verestert oder verethert ist oder
wobei die Alkylengruppe eine durch ein Sauerstoffatom und/oder Stickstoffatom und/oder Schwefelatom und/oder Estergruppen und/oder Thioestergruppen unterbrochene und durch eine Hydroxylgruppe substituierte Kohlenwasserstoffgruppe ist und/oder diese Hydroxylgruppe verestert oder verethert ist und
wobei unterschiedliche Y gleich oder verschieden sein können und
wobei Y 20 oder weniger Kohlenstoffatome enthält und

wobei YZ so gewählt wird, dass Z immer möglichst viele Atome aufweist.

Disiloxane zeichnen sich durch eine außergewöhnliche Kettenbeweglichkeit aus, da die Verbindungen eine freie Rotation um die Silizium-Sauerstoffverbindungen aufweisen, die u.a. auch durch den Größenunterschied zwischen dem Sauerstoffatom und dem Siliziumatom begründet ist. Dies führt zu Verbindungen, die aufgrund der Flexibilität ihres Strukturgerüsts eine auffallend niedrige Viskosität besitzen. Weiterhin führt die Abschirmung der Sauerstoffatome durch die Kohlenwasserstoffgruppen am Silizium zu einer ausgeprägten Hydrophobie und zu einer stark eingeschränkten Wechselwirkung der Ketten untereinander, so dass das Benetzen von Oberflächen begünstigt werden sollte.

Neben einigen kommerziell erhältlichen erfindungsgemäßen Disiloxanen (z.B. 1,3-Bis(3-methacryloxypropyl)tetramethyldisiloxan, 1,3-Bis(3-methacryloxy-2-hydroxypropoxypropyl)tetramethyldisiloxan, 1,3-Bis[(acryloxymethyl)phenethyl]tetramethyldisiloxan) gibt es für die Herstellung der erfindungsgemäßen Disiloxane zahlreiche Synthesestrategien.

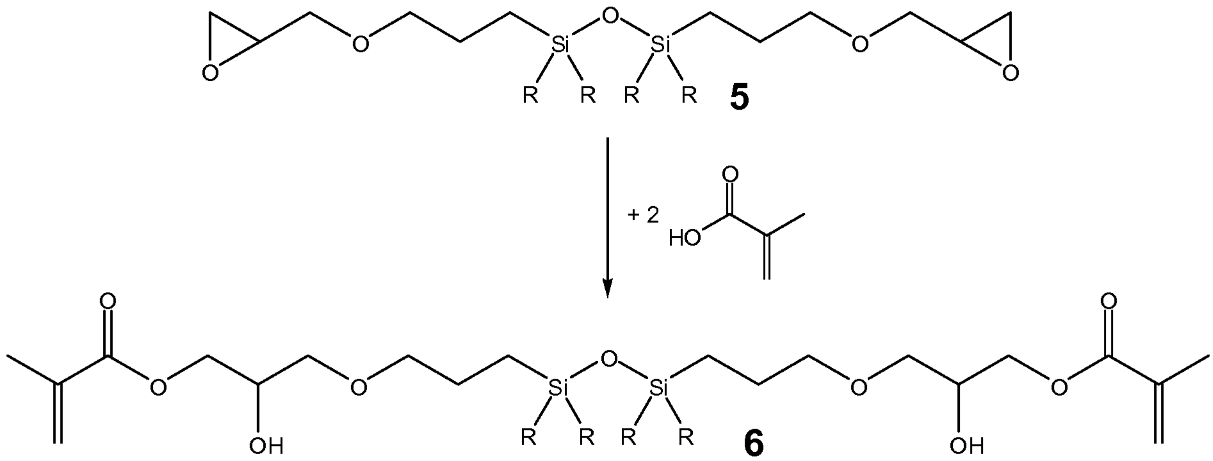


5

6

Das 1,3-Bis(3-methacryloxy-2-hydroxypropoxypropyl)disiloxan **6** lässt sich durch Umsetzung des entsprechenden 1,3-Bis(3-glycidoxypropyl)disiloxans **5** mit Methacrylsäure herstellen.

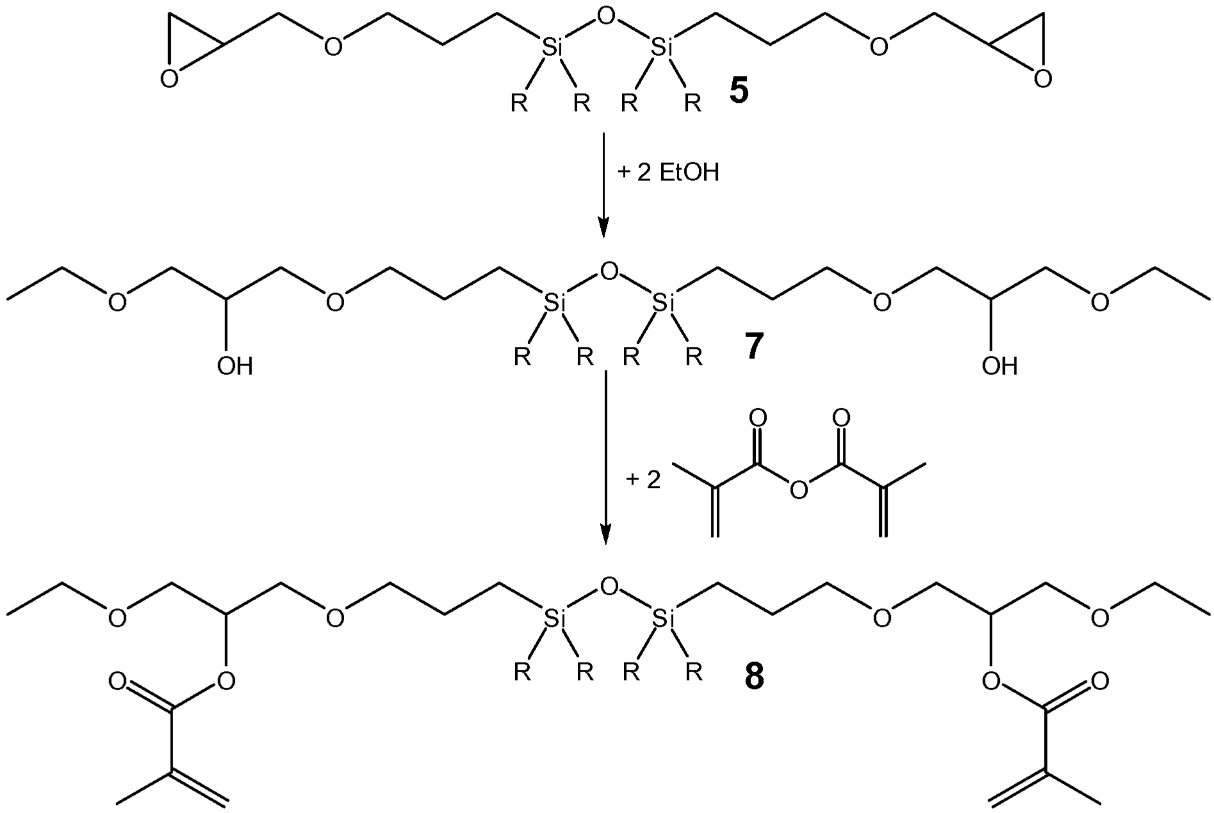


5

7

8

Aus dem 1,3-Bis(3-glycidoxypropyl)disiloxans **5** lässt sich durch Umsetzung mit Ethanol und anschließend Methacrylsäureanhydrid das Disiloxan **8** synthetisieren.

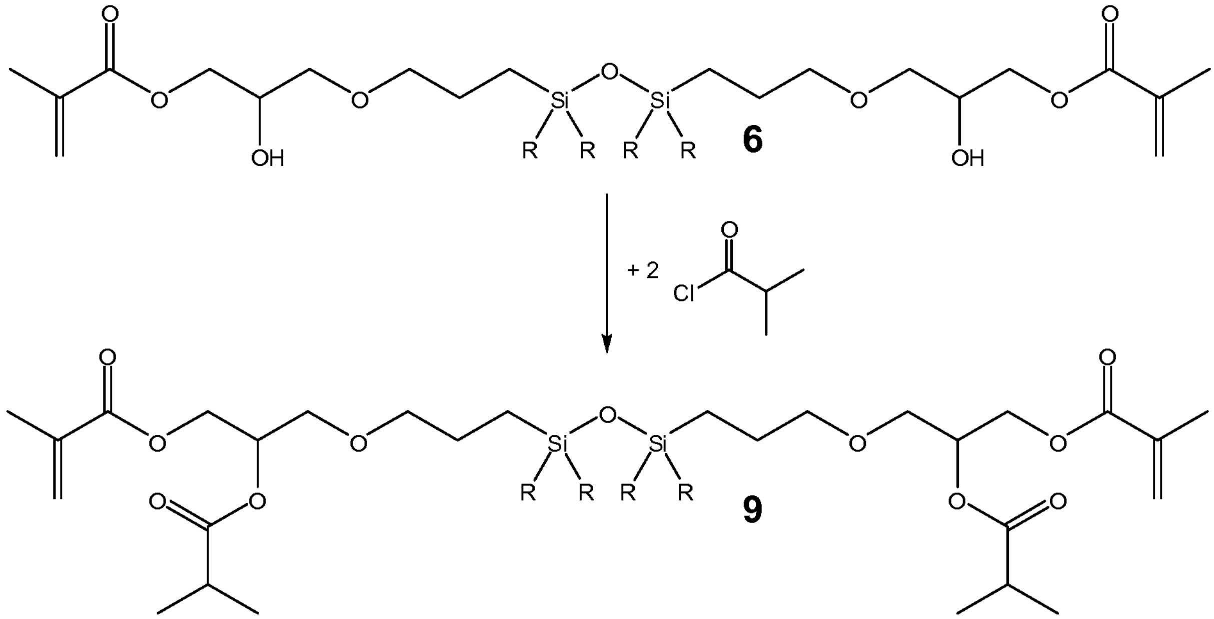


**6**

**9**

Aus dem 1,3-Bis(3-methacryloxy-2-hydroxypropoxypropyl)disiloxan **6** lassen sich durch weitere Umsetzung mit unterschiedlichen Säurechloriden oder -anhydriden die entsprechenden Disiloxane **9**, **10**, **11**, **12** und **13** herstellen.

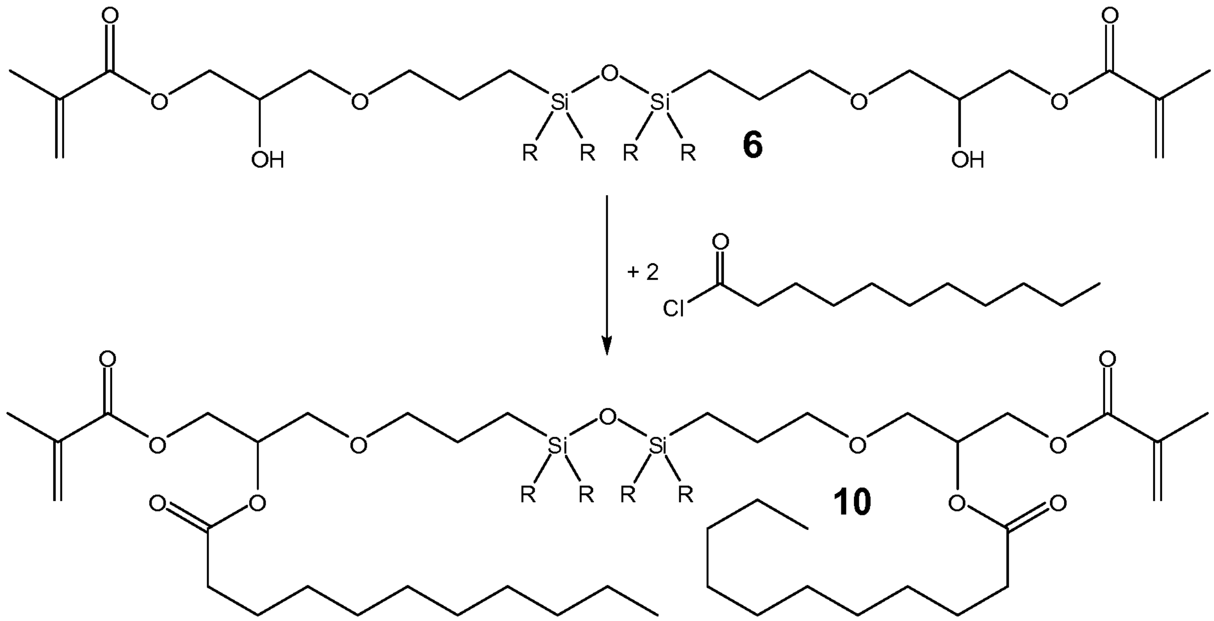


**6**

**10**

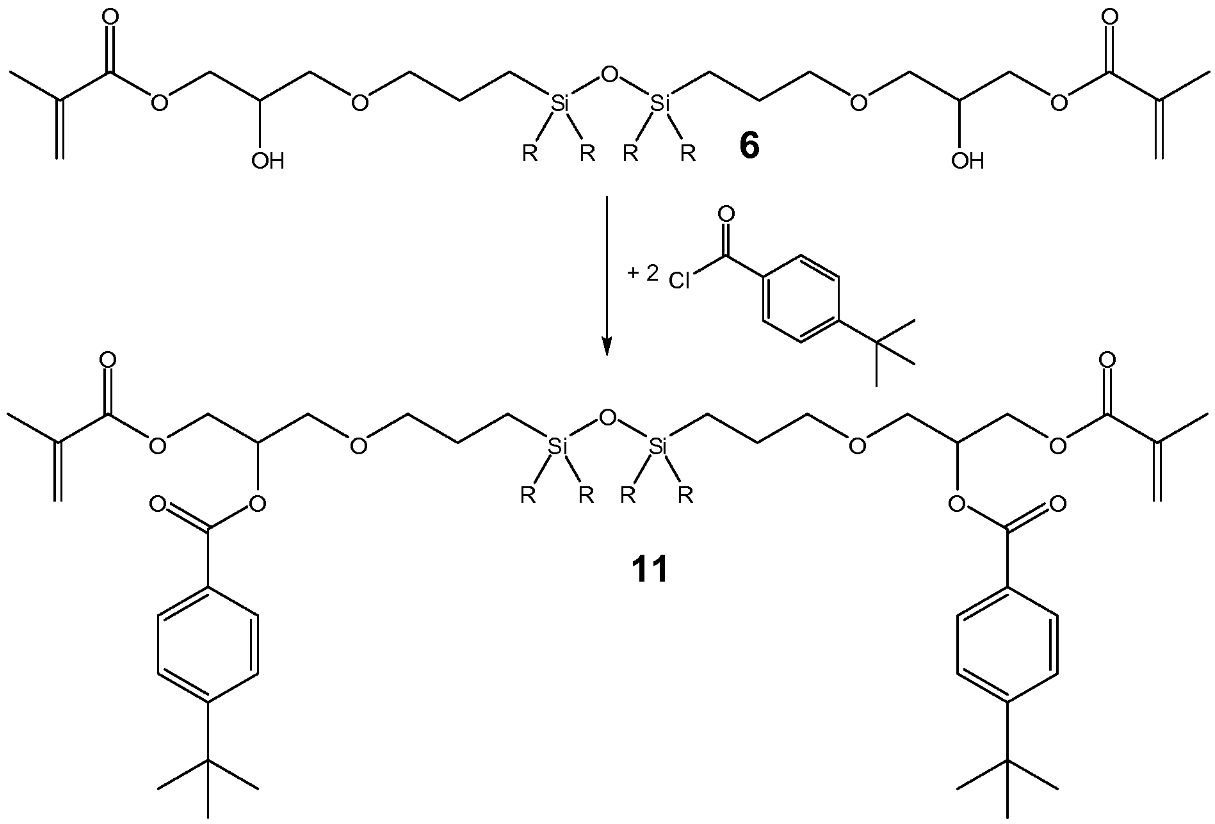


6

11

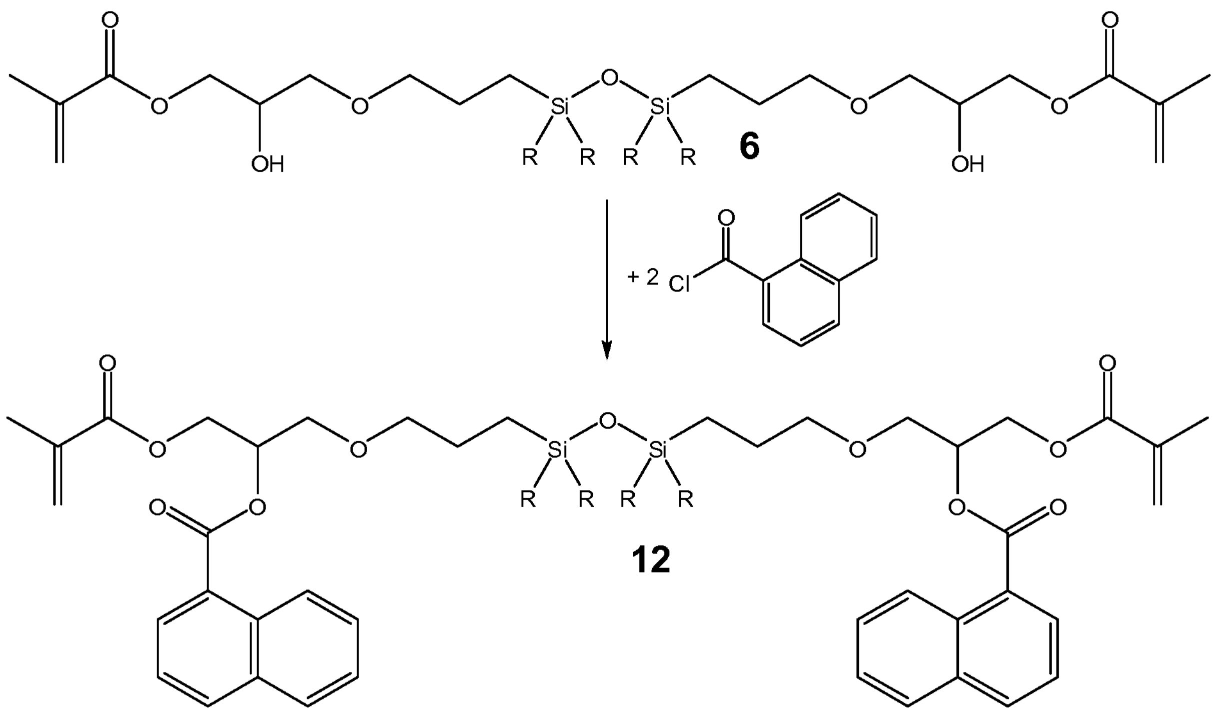


6

12

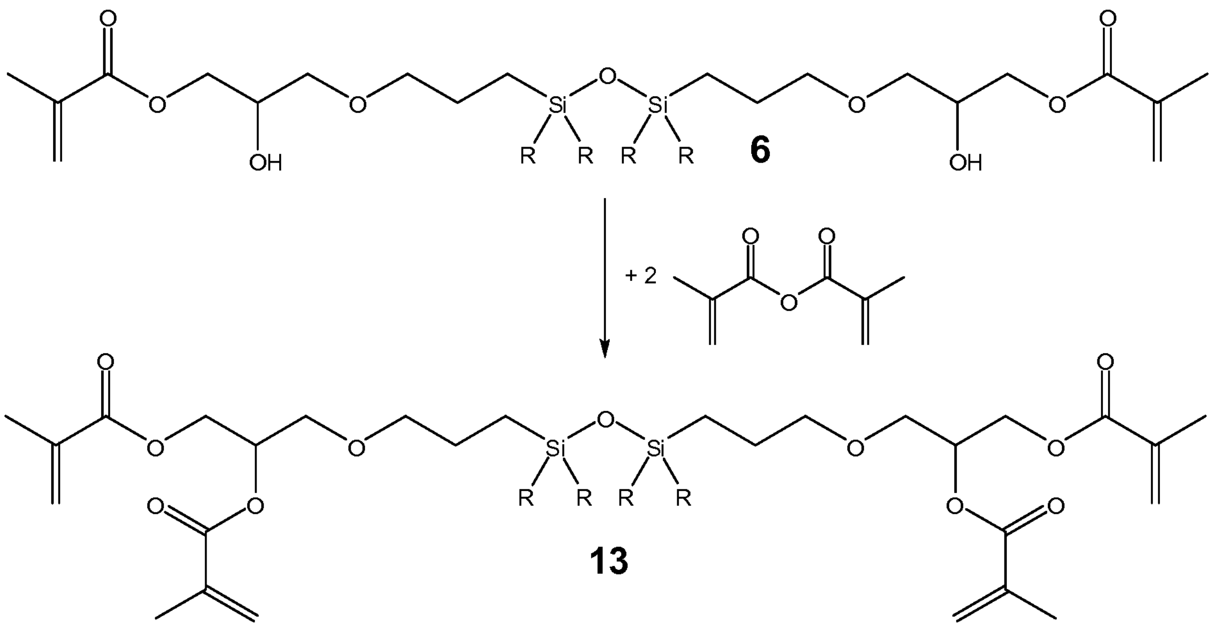


6

13

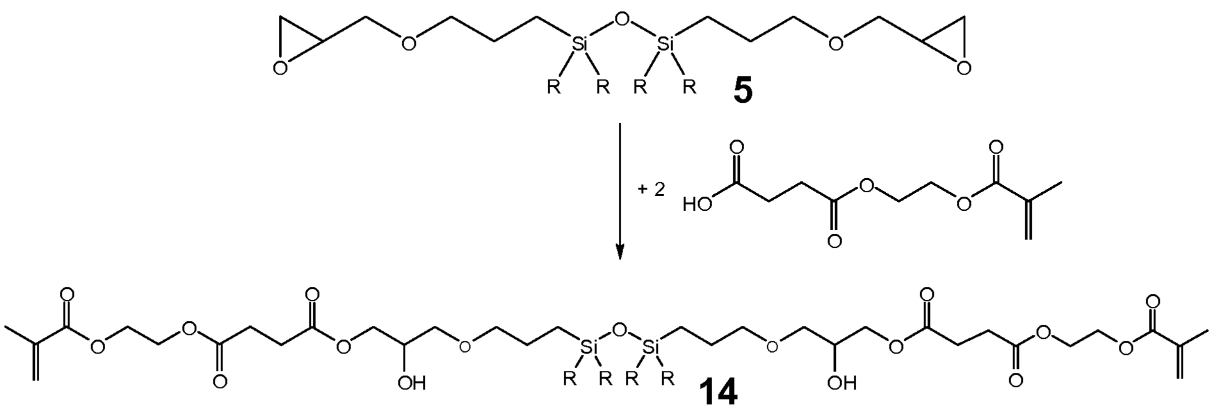


5

14

Aus dem 1,3-Bis(3-glycidoxypropyl)disiloxans **5** lässt sich auch durch Umsetzung mit Bernsteinsäuremono(2-methacryloxyethyl)ester das entsprechende Disiloxan **14** synthetisieren.

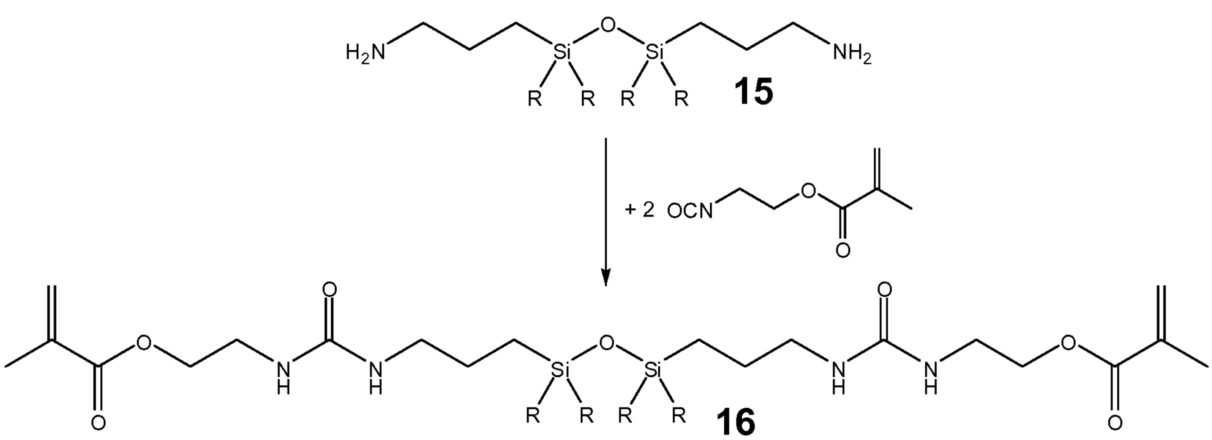


15

16

Aus dem kommerziell erhältlichen 1,3-Bis(3-aminopropyl)disiloxan **15** erhält man durch Umsetzung mit Methacrylsäure-2-isocyanatoethylester das entsprechende Harnstoff-Derivat **16.**

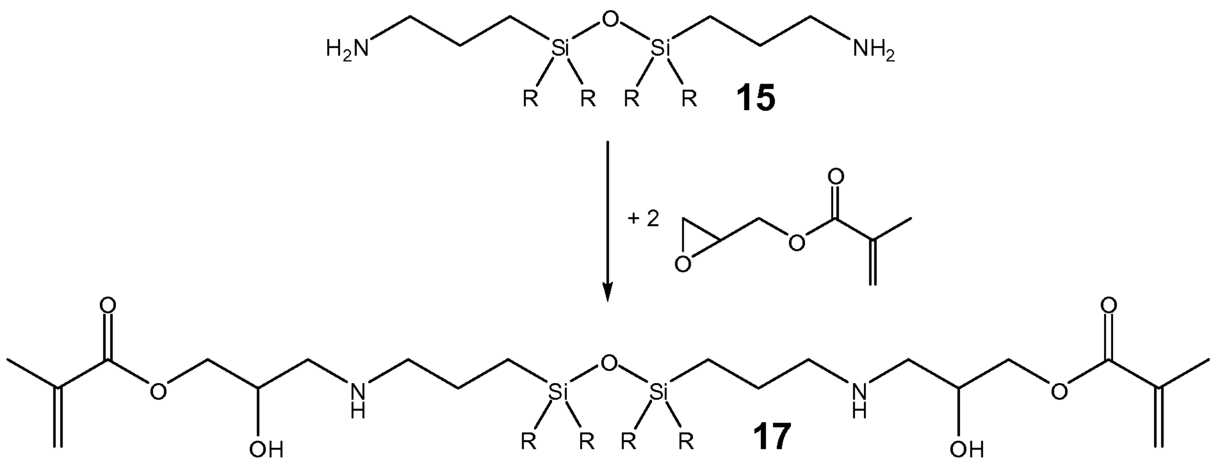


15

17

Aus dem 1,3-Bis(3-aminopropyl)disiloxan **15** lassen sich durch Umsetzung mit 2 bzw. 4 Äquivalenten Methacrylsäureglycidylester die entsprechenden Disiloxane **17** bzw. **18** herstellen.

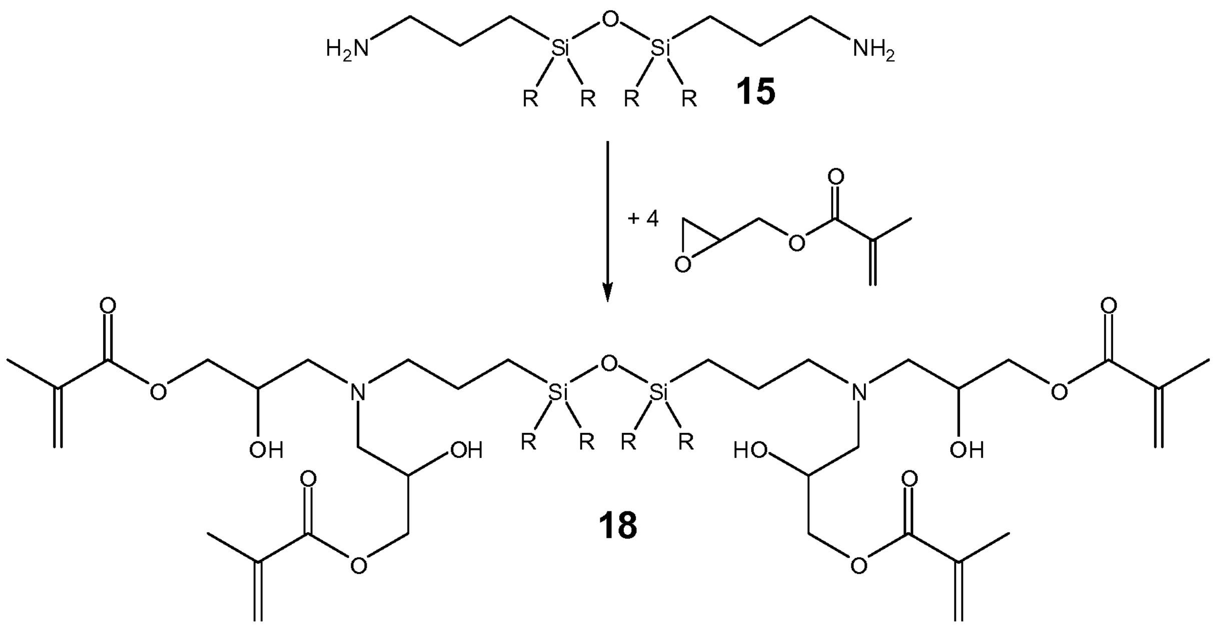


15

18

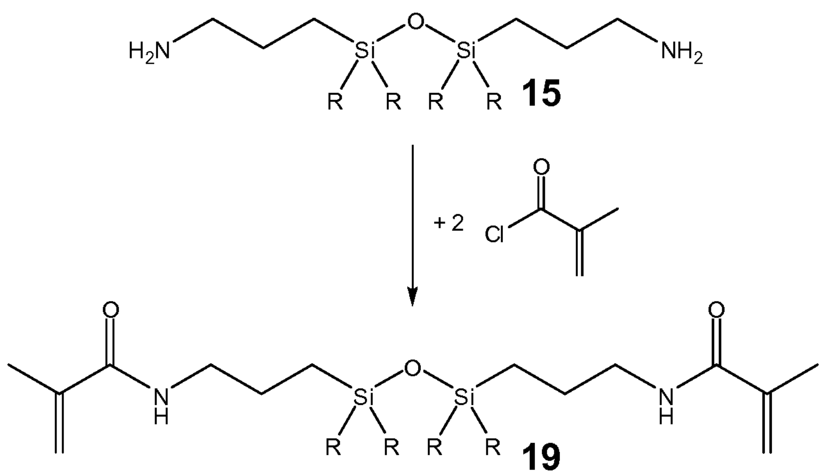


15

19

Ebenfalls aus dem 1,3-Bis(3-aminopropyl)disiloxan **15** ist das 1,3-Bis(3-methacrylamidopropyl)disiloxan 19 durch Umsetzung mit Methacrylsäurechlorid erhältlich.

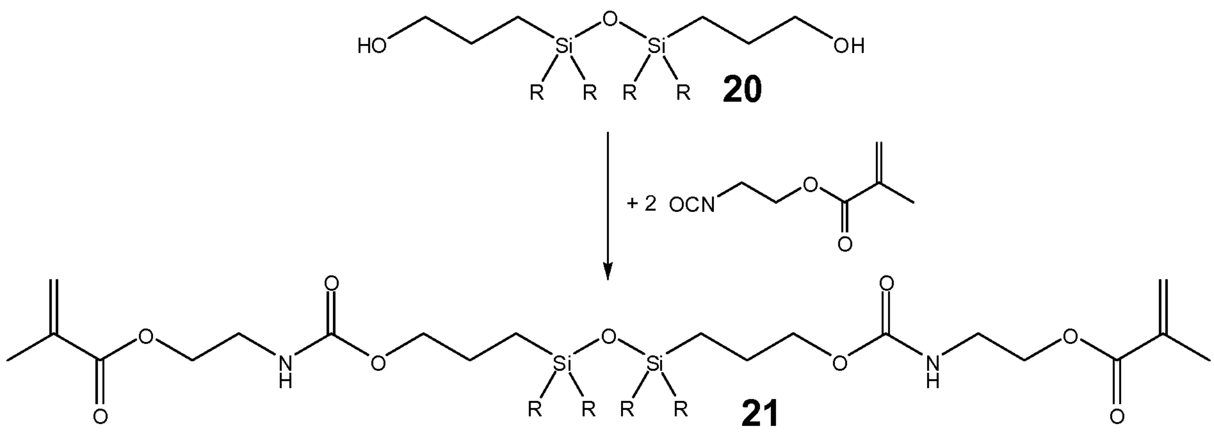


20

21

Aus dem kommerziell erhältlichen 1,3-Bis(3-hydroxypropyl)disiloxan **20** erhält man durch Umsetzung mit Methacrylsäure-2-isocyanatoethylester das entsprechende Urethan-Derivat **21.**

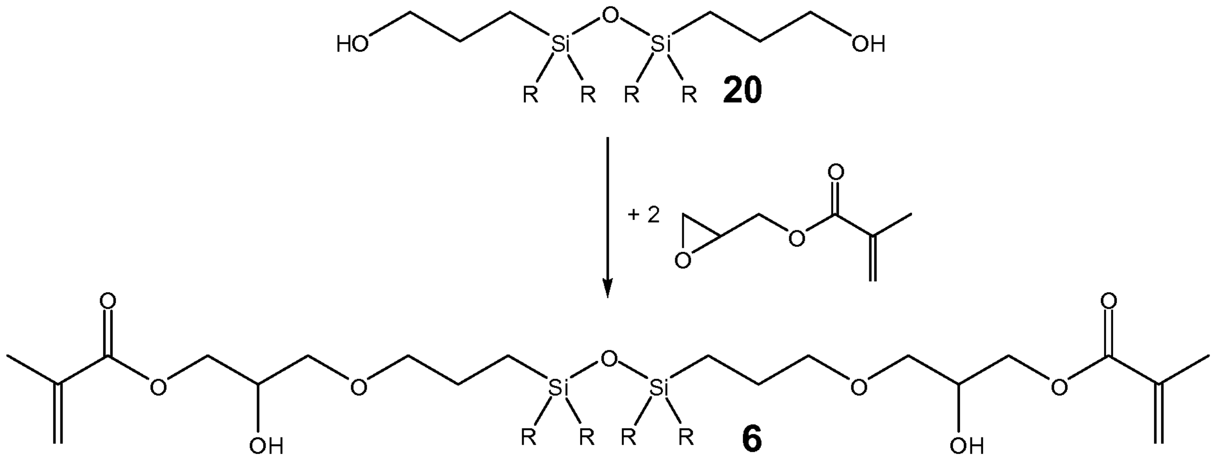


20

6

Aus dem 1,3-Bis(3-hydroxypropyl)disiloxan **20** ist durch Umsetzung mit Methacrylsäureglycidylester auch wiederum das entsprechende 1,3-Bis(3-methacryloxy-2-hydroxypropoxypropyl)disiloxan **6** erhältlich.

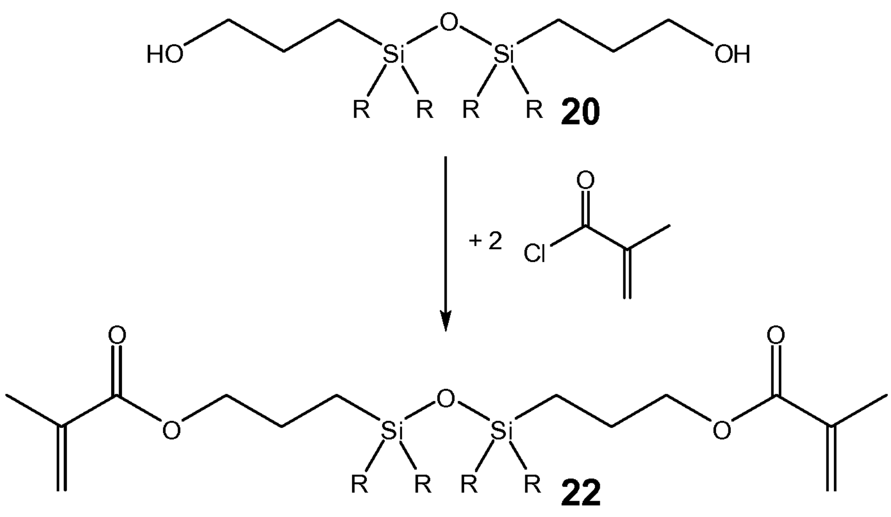


20

22

Ebenfalls aus dem 1,3-Bis(3-hydroxypropyl)disiloxan **20** erhält man durch Umsetzung mit Methacrylsäurechlorid das entsprechende 1,3-Bis(3-methacryloxypropyl)disiloxan **22.**

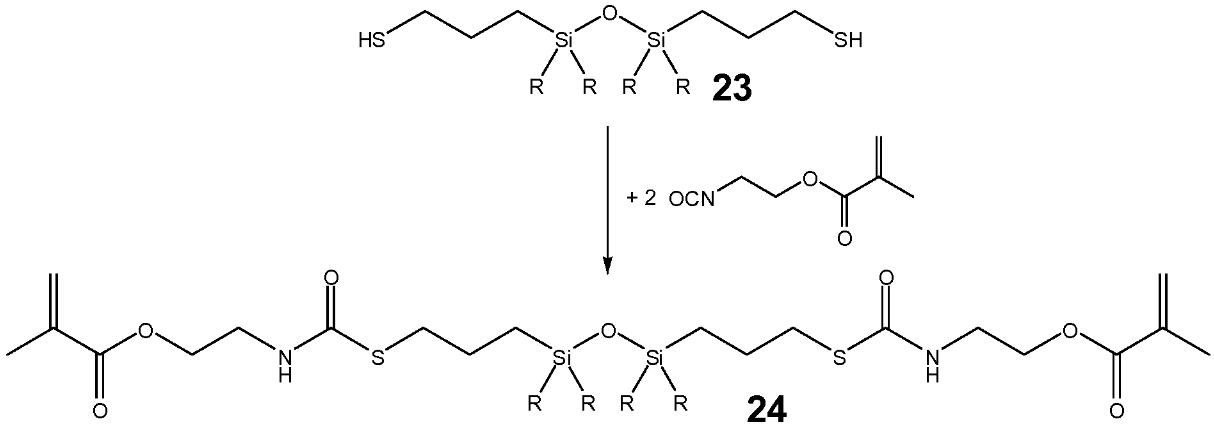


23

24

Aus dem kommerziell erhältlichen 1,3-Bis(3-mercaptopropyl)disiloxan **23** erhält man durch Umsetzung mit Methacrylsäure-2-isocyanatoethylester das entsprechende Thiourethan-Derivat **24.**

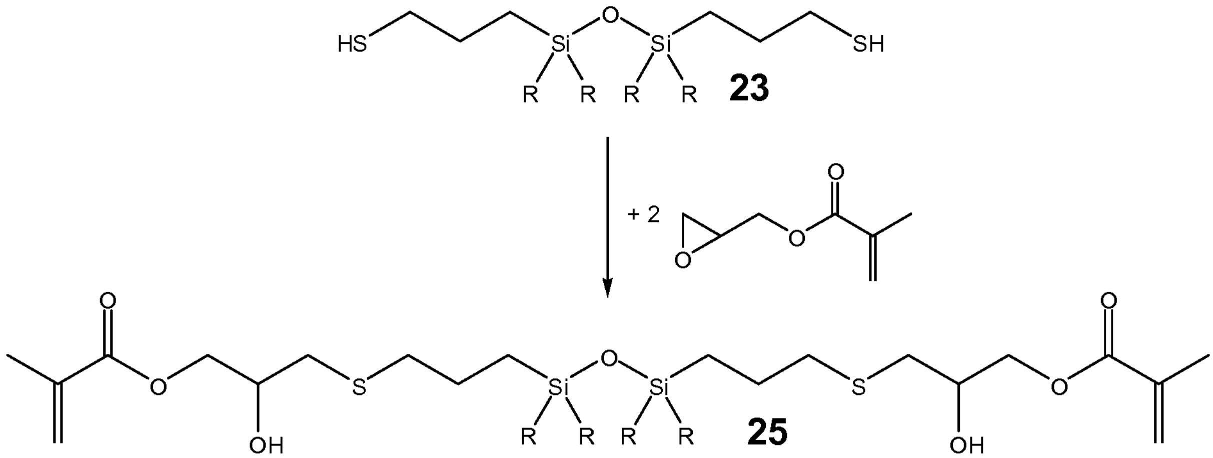


23

25

Aus dem 1,3-Bis(3-mercaptopropyl)disiloxan **23** ist durch Umsetzung mit Methacrylsäureglycidylester auch das entsprechende Disiloxan **25** erhältlich.

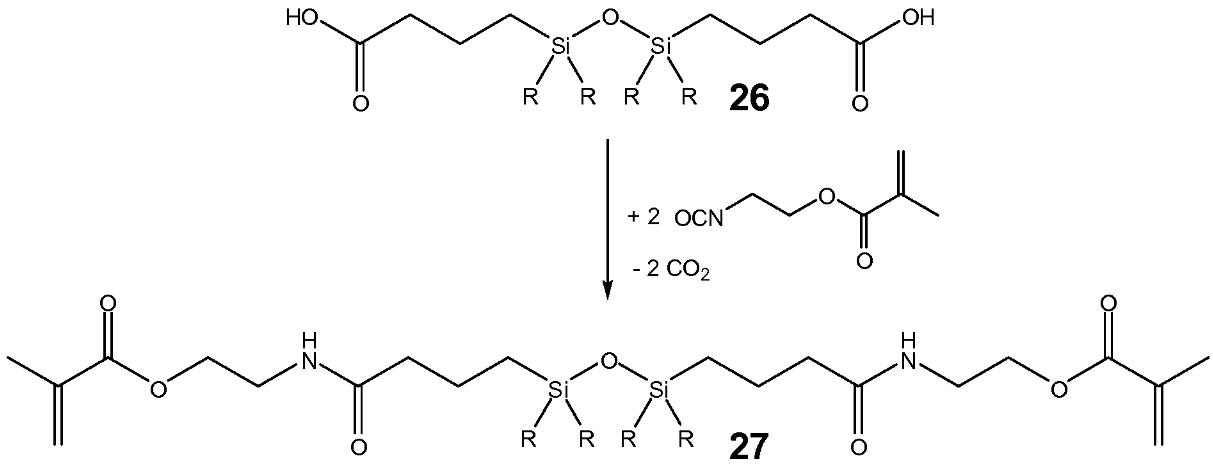


26

27

Aus dem kommerziell erhältlichen 1,3-Bis(3-carboxypropyl)disiloxan 26 erhält man durch Umsetzung mit Methacrylsäure-2-isocyanatoethylester das entsprechende Amid-Derivat 27.

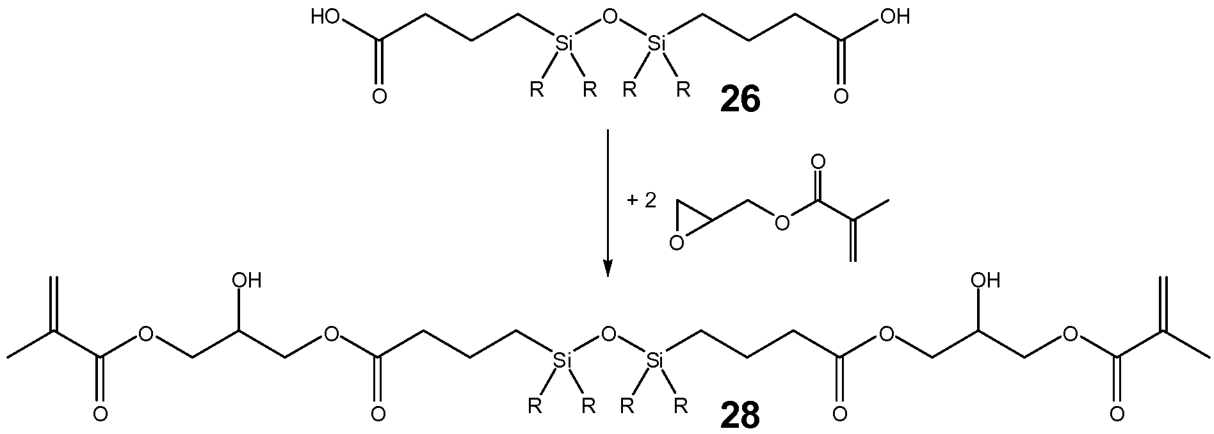


26

28

Aus dem 1,3-Bis(3-carboxypropyl)disiloxan **26** ist durch Umsetzung mit Methacrylsäureglycidylester auch das entsprechende Disiloxan **28** erhältlich.

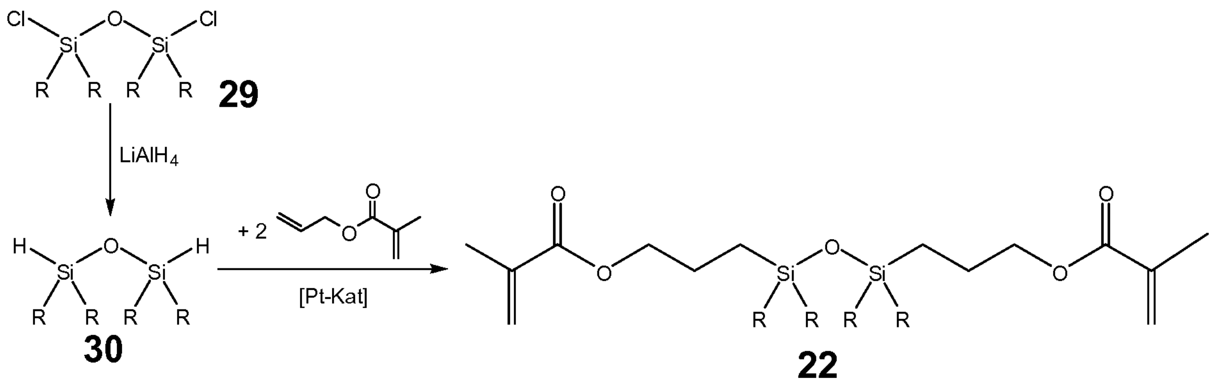


29

30

22

Selbstverständlich sind auch ausgehend von den Dichlorodisiloxanen durch typische Silanchemie eine Vielzahl von (meth)acrylsubstituierten Disiloxanen erhältlich. Beispielhaft sei hier die Umsetzung mit Lithiumaluminiumhydrid zum Dihydrodisiloxan und anschließende Platin-katalysierte Umsetzung mit Allylmethacrylat zum entsprechenden 1,3-Bis(3-methacryloxypropyl)disiloxan **22** erwähnt.

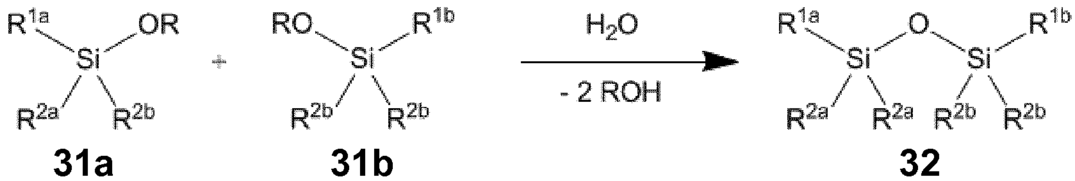


31a 31b 32

Selbstverständlich sind die erfindungsgemäßen Disiloxane nicht nur durch Funktionalisierung von Disiloxanen erhältlich, sondern lassen sich auch ausgehend von Monoalkoxysilanen durch Hydrolyse und Kondensation darstellen. So sind beispielsweise auch die unsymmetrischen Disiloxane **32** durch Umsetzung unterschiedlicher Monoalkoxysilane **31**a/b erhältlich. Für die Funktionalisierung der Monoalkoxysilane sind unter anderem die gleichen Funktionalisierungsreaktionen denkbar, die bereits für die Disiloxane beschrieben wurden.

Bestandteil c.) - optional ein, zwei, drei oder mehrere radikalisch härtbare Monomere ohne Siliziumatom
Die radikalisch härtbaren Monomeren ohne Si-Atom sind Monomere, die bevorzugt eine, zwei oder mehr ethylenische Gruppen aufweisende Substanzen sind, wie beispielsweise, ohne darauf beschränkt zu sein, (Meth)acrylatmonomere.

Die (Meth)acrylatmonomere können monofunktionell sowie polyfunktionell sein.

Vorzugsweise eingesetzte monofunktionelle (Meth)acrylatmonomere stellen die Ester der (Meth)acrylsäure mit Alkylgruppen von 1 bis 12 C-Atomen sowie Ester der (Meth)acrylsäure, die aromatische Gruppen mit 6 bis 12 C-Atomen enthalten, wobei die Alkylgruppen und aromatischen Gruppen, die die Ester bilden, Substituenten wie Hydroxylgruppen und Etherbindungen enthalten können, dar.

In der Patentliteratur ist eine Vielzahl weiterer Verbindungen genannt (beispielsweise auch in der DE 39 41

629 A1, welche im Wege der Verweisung Bestandteil der vorliegenden Anmeldung ist), die allesamt Ester der Acryl- oder Methacrylsäure sind und sich zum Einsatz in einem härtbaren Gemisch eignen.

Die radikalisch polymerisierbaren Monomere können auch mindestens eine ethylenische Doppelbindung aufweisende Hydroxylverbindungen sein. Dabei können vorzugsweise die üblicherweise eingesetzten Hydroxylverbindungen von (Meth)acrylaten eingesetzt werden.

Auch können als Beispiele polyfunktioneller (Meth)acrylatmonomere Di(meth)acrylate von Alkylenglykol mit 2 bis 20 C-Atomen, Di(meth)acrylate von Oligomeren des Alkylenglykols, Polyalkylenglykoldi(meth)acrylat, Di(meth)acrylate von Bisphenol A bzw. vom Diglycidylether von Bisphenol A genannt werden.

Besonders bevorzugt sind weiterhin radikalisch härtbare Verbindungen, die auf einem zentralen polyalicyclischen Strukturelement beruhen, wie beispielsweise 3(4), 8(9)-Bis((meth)acryloyloxymethyl)tricyclo[5.2.1.0$^{2,6}$]decan, alkoxyliertes 3(4), 8(9)-Bis((meth)acryloyloxymethyl)tricyclo[5.2.1.0$^{2,6}$]decan, 2,3-Bis((meth)acryloyloxymethyl)-bicyclo[2.2.1]heptan, alkoxyliertes 2,3-Bis((meth)acryloyloxymethyl)bicyclo[2.2.1]heptan, 1,3,5-Tri(meth)acryloyloxytricyclo[3.3.1.1$^{3,7}$]decan, alkoxyliertes Tri(meth)acryloyloxytricyclo-[3.3.1.1$^{3,7}$]decan sowie (Meth)acrylsäureester von Tricyclo[5.2.1.0$^{2,6}$]decan-3(4),8(9)-dimethanol, alkoxyliertem Tricyclo[5.2.1.0$^{2,6}$]decan-3(4),8(9)-dimethanol, Bicyclo[2.2.1]heptan-2,3-dimethanol, alkoxyliertem Bicyclo[2.2.1]heptan-2,3-dimethanol, 1,3,5-Adamantantriol, alkoxyliertem 1,3,5-Adamantantriol, wobei zwischen dem polyalicyclischen Strukturelement und den (Meth)acrylsäureestern Urethan-, Harnstoff-, Amid-, Allophanat-, Acylharnstoff- oder Biuretgruppen angeordnet sind.

Angaben zur Herstellung dieser substituierten (Meth)acrylsäureester finden sich in den Patentanmeldungen EP 11 183 333, EP 11 183 328, EP 11 183 345, EP 11 183 338, EP 11 183 342 und EP 11 188 086 sowie in den in diesen Dokumenten zitierten Schriftstücken. Diese Literaturstellen sind im Wege der Verweisung ebenfalls Bestandteil der vorliegenden Anmeldung.

Bevorzugt sind ebenfalls Urethan(meth)acrylatester, Reaktionsprodukte aus 2 Mol eines (Meth)acrylats mit einer Hydroxylgruppe und einem Mol eines Diisocyanats.

In einem bevorzugten erfindungsgemäßen härtbaren Gemisch enthält Bestandteil (c) ein oder mehrere (Meth)acrylat-Monomere gewählt aus der Gruppe bestehend aus 3(4), 8(9)-Bis((meth)acryloyloxymethyl)tricyclo[5.2.1.0$^{2,6}$]decan, alkoxyliertem 3(4), 8(9)-Bis((meth)acryloyloxymethyl)tricyclo[5.2.1.0$^{2,6}$]decan, 2,3-Bis((meth)acryloyloxymethyl)-bicyclo[2.2.1]heptan, alkoxyliertem 2,3-Bis((meth)acryloyloxymethyl)bicyclo[2.2.1]heptan, 1,3,5-Tri(meth)acryloyloxytricyclo[3.3.1.1$^{3,7}$]decan, alkoxyliertem Tri(meth)acryloyloxytricyclo-[3.3.1.1$^{3,7}$]decan, (Meth)acrylsäureester von Tricyclo[5.2.1.0$^{2,6}$]decan-3(4),8(9)-dimethanol, alkoxyliertem Tricyclo[5.2.1.0$^{2,6}$]decan-3(4),8(9)-dimethanol, Bicyclo[2.2.1]heptan-2,3-dimethanol, alkoxyliertem Bicyclo[2.2.1]heptan-2,3-dimethanol, 1,3,5-Adamantantriol, alkoxyliertem 1,3,5-Adamantantriol, wobei zwischen dem polyalicyclischen Strukturelement und den (Meth)acrylsäureestern Urethan-, Harnstoff-, Amid-, Allophanat-, Acylharnstoff- oder Biuretgruppen angeordnet sind, Ethylenglykoldi(meth)acrylat, 1,6-Hexandioldi(meth)acrylat (HEDMA), Triethylenglykoldi(meth)acrylat (TEGDMA), 1,12-Dodecandioldi(meth)acrylat, Bisphenol-A-di(meth)acrylat, alkoxyliertes Bisphenol-A-di(meth)acrylat, Bisphenol-B-di(meth)acrylat, alkoxyliertes Bisphenol-B-di(meth)acrylat, Bisphenol-C-di(meth)acrylat, alkoxyliertes Bisphenol-C-di(meth)acrylat, Bisphenol-F-di(meth)acrylat, alkoxyliertes Bisphenol-F-di(meth)acrylat, Polyethylenglykoldi(meth)acrylat, 7,7,9-Trimethyl-4,13-dioxo-5,12-diazahexadecan-1,16-dioxydi(meth)acrylat (UDMA), Butandioldi(meth)acrylat, Tetraethylenglykoldi(meth)acrylat, Neopentylglykoldi(meth)acrylat, 2-Hydroxypropyl-1,3-di(meth)acrylat, 3-Hydroxypropyl-1,2-di(meth)acrylat, Pentaerythritoldi(meth)acrylat, Di(meth)acrylaten des Dihydroxymethyltricyclo[5.2.1.0$^{2,6}$]decans, 2-Hydroxyethyl(meth)acrylat, 2-Hydroxypropyl(meth)acrylat, 3-Hydroxypropyl(meth)acrylat, 1,2-Dihydroxypropyl(meth)acrylat, 1,3-Dihydroxypropyl(meth)acrylat, 2,2-Bis[4-[3-(meth)acryloyloxy-2-hydroxypropoxy]phenyl]propan (Bis-GMA), Trimethylolpropantri(meth)acrylat, Trimethylolethantri(meth)acrylat, Pentaerythritoltri(meth)- acrylat, Trimethylolmethantri(meth)acrylat, Pentaerythritoltetra(meth)acrylat, Ditrimethylolpropantetra(meth)acrylat, Pentaerythritolhexa(meth)acrylat, Butylenglykoldi(meth)acrylat, Propylenglykoldi(meth)acrylat, Nonandioldi(meth)acrylat, Decandioldi(meth)acrylat, Glycerolmono(meth)acrylat, Glyceroldi(meth)acrylat, Trimethylolpropanmono(meth)acrylat, Trimethylolpropandi(meth)acrylat, Sorbitolmono-, di-, tri-, tetra- oder penta(meth)acrylat, Methyl(meth)acrylat, Ethyl(meth)acrylat, Propyl(meth)acrylat, Butyl(meth)acrylat, Hexyl(meth)acrylat, Tetrahydrofurfuryl(meth)acrylat, Lauryl(meth)acrylat, Cyclohexyl(meth)-acrylat, Allyl(meth)acrylat, Glycidyl(meth)acrylat, 2-Ethoxyethyl(meth)acrylat, Methoxypolyethylenglykol(meth)acrylat, Isobornyl(meth)acrylat, 2-(N,N-Dimethylamino)ethyl(meth)acrylat, N-Methylol(meth)acrylamid, Diaceton(meth)acrylamide, 2,2-Bis[4-(meth)acryloyloxyphenyl]-propan, 2,2-Bis[4-(meth)acryloyloxyethoxyphenyl]propan, 2,2-Bis[4-(meth)acryloyloxydiethoxyphenyl]propan, 2,2-Bis[4-(meth)acryloyloxytriethoxyphenyl]propan 2,2-Bis[4-(meth)acryloyloxytetraethoxyphenyl]propan, 2,2-Bis[4-(meth)acryloyloxypentaethoxyphenyl]-propan, 2,2-Bis[4-(meth)acryloyloxydipropoxyphenyl]propan, 2,2-Bis[4-(meth)acryloyloxyethoxyphenyl]-2-[4-(meth)acryloyloxydiethoxyphenyl]propan, 2-[4-(Meth)acryloyloxydiethoxyphenyl]-2-[4-(meth)acryloyloxytriethoxyphenyl]propan, 2-[4-(Meth)acryloyloxdipropoxy- phenyl]-2-[4-(meth)acryloyloxytriethoxyphenyl]propan, 2,2-Bis[4-(meth)acryloyloxyisopropoxy phenyl]propan, Hydroxypivalinsäureneopentylglykoldi(meth)acrylat, Acetoacetoxyethyl-(meth)acrylat, Polypropylenglykoldi(meth)acrylat, Glycerolalkoxylatdimethacrylat, Neopentylglykol(meth)acrylat, N,N-(1,2-Dihydroxyethylen)bisacrylamid, 2,2-Bis[4-(meth)acryloyloxypentaethoxyphenyl]propan, 2,2-Bis[4-(meth)acryloyloxypolyethoxyphenyl]-propan, Diethylenglykoldi(meth)acrylat, Dipentaerythritoltetra(meth)acrylat, Dipentaerythritolhexa(meth)acrylat, N,N-(2,2,4-Trimethylhexamethylen)bis[2-(aminocarboxy)propan-1,3-diol]-tetra(meth)acrylat, das Kondensationsprodukt von 3, (4)- (Meth)acryloxymethyl-8, (9) - hydroxymethyltricyclo- [5.2.1.0$^{2,6}$]decan mit Dicarbonsäuren, 2-Ethylhexyl(meth)acrylat, Tridecyl(meth)acrylat, Stearyl(meth)acrylat, Benzyl(meth)acrylat, Methoxydiethylenglykol(meth)acrylat, Dicyclopentenyl(meth)acrylat, Phenyl(meth)acrylat, Pentaerythritolmono(meth)acrylat, Dipentaerythritolmono(meth)acrylat, Caprolacton modifiziertes Tetrahydrofurfuryl(meth)acrylat.

Bestandteil d.) - Füllstoffe

Eine erfindungsgemäße radikalisch härtbare dentale Dentalzusammensetzung enthält einen Anteil an Füllstoffpartikeln von 85 Gew.-% oder weniger, vorzugsweise von 78 Gew.-% oder weniger, bezogen auf die Gesamtmasse der erfindungsgemäßen Dentalzusammensetzung.

Als Bestandteil (d.) können organische und/oder anorganische Füllstoffe eingesetzt werden.

Organische Füllstoffpartikel umfassen oder bestehen aus beispielsweise ein oder mehr Verbindungen ausgewählt aus der Gruppe bestehend aus Polyvinylacetat und Copolymere des Polyvinylacetats mit einer oder mehreren polymerisierbaren Verbindungen, Polystyrol, Polyethylen, Polypropylen, Wachse wie Polyethylenwachs, Polybutylen, Polybutadien, Copolymere des Butadiens und des Styrols, Polyacrylnitril, Harze wie Kolophoniumharz oder Kohlenwasserstoffharze, Poly(meth)acrylatester, d.h. Umsetzungsprodukte von Poly(meth)acrylsäure mit linearen oder verzweigten aliphatischen, aromatischen oder cycloaliphatischen Alkoholen wie Methanol, Ethanol, Propanol, Isopropanol, den isomeren Butanolen und höheren Homologen der genannten Alkohole mit bis zu 22 Kohlenstoffatomen, Cyclohexanol, Benzylalkohol und dergleichen, Polydialkylmaleinate wie Dibutylmaleinat und deren Copolymere und Silylgruppen-haltige Polymere wie Polyvinylsilane oder Copolymere von Vinylsilan mit einem oder mehreren der genannten Monomeren. Die organischen Füllstoffe können alleine oder als Mischungen eingesetzt werden.

Die anorganischen Füllstoffe können ebenfalls allein oder als Mischungen eingesetzt werden. Zur Optimierung der Produkteigenschaften können die anorganischen Füllstoffe in unterschiedlichen Korngrößen in die Rezepturen eingebracht werden. Die Füllstoffe können eine unimodale oder polymodale, beispielsweise eine bimodale Verteilung aufweisen.

Als anorganische Füllstoffe können kompakte Gläser und unterschiedliche Kieselsäuren in verschiedenen Größen und Zuständen (monodispers, polydispers) zum Einsatz kommen.

Geeignete anorganische Bestandteile sind beispielsweise amorphe Materialien auf der Basis von Mischoxiden aus $SiO_2$, $ZrO_2$ und/oder $TiO_2$ sowie Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silizium, Zeolithe, Apatit, Zirkonsilikate, schwerlösliche Metallsalze wie Bariumsulfat oder Calciumfluorid sowie röntgenopake Füllstoffe wie Ytterbiumfluorid.

Zum besseren Einbau in die Polymermatrix können die Füllstoffe organisch oberflächenmodifiziert sein. Beispielhaft genannt sei die Oberflächenbehandlung der Füllstoffe mit einem Silan. Als Haftvermittler eignet sich besonders das Methacryloxypropyltrimethoxysilan.

Zur Einstellung der Rheologie können die radikalisch härtbaren Zusammensetzungen unterschiedliche Kieselsäuren, bevorzugt pyrogene Kieselsäuren, enthalten.

Bevorzugt enthalten die erfindungsgemäßen härtbaren Zusammensetzungen nanoskalige Feststoffteilchen. Bei den nanoskaligen Feststoffteilchen handelt es sich um Partikel mit einer mittleren Teilchengröße von nicht mehr als 200 nm, bevorzugt nicht mehr als 100 nm und insbesondere nicht mehr als 70 nm. Die nanoskaligen anorganischen Feststoffteilchen sind bevorzugt solche von Oxiden, Sulfiden, Seleniden und Telluriden von Metallen, Mischmetallen und deren Mischungen. Besonders bevorzugt sind nanoskalige Teilchen von $SiO_2$, $TiO_2$, $ZrO_2$, ZnO, $SnO_2$ und $Al_2O_3$ und deren Mischungen. Die Herstellung der nanoskaligen Feststoffteilchen erfolgt auf bekannte Weise, z.B. durch Flammpyrolyse, Plasmaverfahren, Gasphasenkondensation, Kolloidtechniken, Präzipitationsverfahren, Sol-Gel Verfahren, etc.

In einer bevorzugten Ausgestaltung liegen die nanoskaligen Teilchen in nicht agglomerierter und/oder nicht aggregierter Form vor, beispielsweise dispergiert in einem Medium, vorzugsweise in monodisperser Form.

Um eine gute Einbindung der Nanopartikel in die Polymermatrix einer erfindungsgemäßen radikalisch härtbaren dentalen Zusammensetzung zu ermöglichen, sind die Oberflächen der Nanopartikel ebenfalls organisch oberflächenmodifiziert, d.h. ihre Oberflächen weisen organische Strukturelemente auf. Beispielhaft genannt sei die Oberflächenbehandlung der Füllstoffe mit einem Silan. Als Haftvermittler eignet sich auch hier besonders das Methacryloxypropyltrimethoxysilan.

In einer weiteren bevorzugten Ausgestaltung sind die nanoskaligen Teilchen somit nicht agglomerierte und/oder nicht aggregierte, organisch oberflächenmodifizierte Nanopartikel mit einer mittleren Partikelgröße kleiner 200 nm, vorzugsweise kleiner 100 nm, besonders bevorzugt kleiner 70 nm, die wiederum bevorzugt silanisiert sind.

Kommerziell erhältliche nanoskalige, nicht-agglomerierte und nicht aggregierte Kieselsole, die erfindungsgemäß eingesetzt werden können, sind beispielsweise unter der Bezeichnung "NALCO COLLOIDAL SILICAS" (Nalco Chemical Co.), "Ludox colloidal silica" (Grace) oder "Highlink OG (Clariant) im Handel.

In einer bevorzugten Ausgestaltung umfasst der Füllstoffanteil einer erfindungsgemäßen radikalisch härtbaren Zusammensetzung eine Mischung eines ersten Füllstoffs d.)1 in Form nicht agglomerierter, nicht aggregierter organisch oberflächenmodifizierter Nanopartikel mit einer mittleren Partikelgröße kleiner 200 nm und eines zweiten Füllstoffs d.)2 in Form von Mikropartikeln mit einer mittleren Partikelgröße im Bereich von 0,4 μm bis 10 μm. Durch die Kombination von d.)1 Nanopartikeln und d.)2 Mikropartikeln in einer erfindungsgemäßen radikalisch härtbaren Zusammensetzung wird eine vollständige und gleichmäßige Volumenfüllung des Kompositmaterials erzielt. Dadurch wird sowohl die Schrumpfung der radikalisch härtbaren Zusammensetzung beim Aushärten der Polymermatrix als auch die Empfindlichkeit der erfindungsgemäßen Zusammensetzung gegen Abrasion vermindert.

Der Anteil organisch oberflächenmodifizierter Nanopartikel in einer bevorzugten erfindungsgemäßen radikalisch härtbaren Zusammensetzung mit einer mittleren Partikelgröße kleiner 200 nm ist größer als 1 Gew.-%, vorzugsweise größer als 2 Gew.-% und besonders bevorzugt größer als 3 Gew.-%. In eigenen Untersuchungen hat sich gezeigt, dass bei einem Gehalt von 1 Gew.-% oder weniger an nicht agglomerierten und/oder nicht aggregierten, organisch oberflächenmodifizierten Nanopartikeln mit einer mittleren Partikelgröße kleiner 200 nm die radikalisch härtbare Zusammensetzung im Einzelfall nicht mehr ausreichend abriebfest ist. Dies ist wahrscheinlich u.a. darauf zurückzuführen, dass bei einem Gehalt von 1 Gew.-% oder weniger an den besagten Nanopartikeln die Hohlräume zwischen den Mikropartikeln mit einer mittleren Partikelgröße von 0,4 μm bis 10 μm nicht mehr ausreichend gefüllt sind. Andererseits hat sich gezeigt, dass bei einem Gehalt von mehr als 50 Gew.-% an nicht agglomerierten und/oder aggregierten, organisch oberflächenmodifizierten Nanopartikeln mit einer mittleren Partikelgröße kleiner 200 nm die Verarbeitbarkeit der Zusammensetzung nicht mehr ausreichend ist. Aufgrund des hohen Feststoffgehalts wird dann seine Viskosität zu hoch.

Die Materialien für die erfindungsgemäß einzusetzenden Nanopartikel sind vorzugsweise Oxide oder Mischoxide und bevorzugt ausgewählt aus der Gruppe bestehend aus Oxiden und Mischoxiden der Elemente Silizium, Titan, Yttrium, Strontium, Barium, Zirkonium, Hafnium, Niob, Tantal, Wolfram, Wismut, Molybdän, Zinn, Zink, Ytterbium, Lanthan, Cer, Aluminium und deren Mischungen. Die bevorzugten oxidischen Nanopartikel sind dabei, wie dargelegt, nicht agglomeriert und/oder nicht aggregiert sowie organisch oberflächenbehandelt.

Innerhalb einer erfindungsgemäßen radikalisch härtbaren Zusammensetzung bewirken die Mikropartikel eine weitgehende gleichmäßige Füllung des Volumens, wobei die verbleibenden Hohlräume zwischen den Mikropartikeln durch die oben beschriebenen Nanopartikel (Komponente d.)1) zumindest teilweise gefüllt werden. Unter Mikropartikeln werden in Zusammenhang mit der vorliegenden Erfindung Partikel mit einer mittleren Partikelgröße von 400 nm bis 10 μm verstanden. Vorzugsweise ist die mittlere Partikelgröße kleiner als 5 μm. Es hat sich gezeigt, dass die mit den Mikropartikeln bereits erreichbare Volumenfüllung der radikalisch härtbaren Zusammensetzung umso vollständiger und gleichmäßiger ist, je kleiner die Mikropartikel sind.

Die Mikropartikel der Komponente d.)2 können eine monomodale oder polymodale, beispielsweise eine bimodale Partikelgrößenverteilung aufweisen. Mikropartikel mit einer bimodalen oder multimodalen Partikelgrößenverteilung sind erfindungsgemäß bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Hohlräume zwischen den Partikeln der Fraktionen mit der größeren Partikelgröße ausfüllen werden. Die noch verbleibenden Hohlräume werden wie oben beschrieben durch Nanopartikel gefüllt.

Ganz besonders bevorzugt wird somit in einer erfindungsgemäßen radikalisch härtbaren Zusammensetzung eine Komponente d.)2 eingesetzt, welche zwei oder mehr Fraktionen von Mikropartikeln enthält, wobei sich die mittleren Partikelgrößen der Fraktionen unterscheiden.

Vorzugsweise enthält Komponente d.)2 zumindest zwei Mikropartikel-Fraktionen, wobei deren mittlere Partikelgrößen um mindestens 0,5 μm, bevorzugt um mindestens 0,7 μm, voneinander abweichen. In manchen Ausgestaltungen beträgt die Differenz der mittleren Partikelgrößen der Mikropartikel-Fraktionen mindestens 1,0 μm.

Die Mikropartikel verschiedener Fraktionen können aus dem gleichen oder aus verschiedenen Materialien bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unterscheiden.

Besonders bevorzugt umfasst eine erfindungsgemäße radikalisch härtbare Zusammensetzung eine Komponente d.)2, welche eine oder mehrere erste Mikropartikelfraktionen, die jeweils eine mittlere Partikelgröße im Bereich von 1 μm bis 10 μm, vorzugsweise 1 μm bis 5 μm, besitzen, und eine oder mehrere zweite Mikropartikelfraktionen, die jeweils eine mittlere Partikelgröße im Bereich von > 0,4 μm bis < 1 μm (d.h. größer als 0,4 μm, aber kleiner als 1 μm), vorzugsweise 0,5 μm bis 0,8 μm, besitzen.

Bevorzugt liegt das Verhältnis der Gesamtmasse der ersten Mikropartikelfraktionen zur Gesamtmasse der zweiten Mikropartikelfraktionen im Bereich von 1 : 1 bis 12 : 1, vorzugsweise im Bereich von 1,5 : 1 bis 8 : 1.

Bevorzugt liegt das Verhältnis der mittleren Korngröße der oder einer ersten Mikropartikelfraktion zur mittleren Korngröße der oder einer zweiten Mikropartikelfraktion der Komponente d.)2 im Bereich von 1,5 : 1 bis 10 : 1, vorzugsweise im Bereich von 2 : 1 bis 5 : 1.

In einer besonders bevorzugten erfindungsgemäßen radikalisch härtbaren Zusammensetzung umfasst die Komponente d.)2 eine oder mehrere erste Mikropartikelfraktionen, die jeweils eine mittlere Partikelgröße im Bereich von 1 μm bis 10 μm, vorzugsweise 1 μm bis 5 μm, besitzen, und eine oder mehrere zweite Mikropartikelfraktionen, die jeweils eine mittlere Partikelgröße im Bereich von > 0,4 μm bis < 1 μm, vorzugsweise 0,5 μm bis 0,8 μm, besitzen; wobei das Verhältnis der Gesamtmasse der ersten Mikropartikelfraktionen zur Gesamtmasse der zweiten Mikropartikelfraktionen im Bereich von 1 : 1 bis 12 : 1, vorzugsweise 1,5 : 1 bis 8 : 1 und/oder das Verhältnis der mittleren Korngröße der oder einer ersten Mikropartikelfraktion zur mittleren Korngröße der oder einer zweiten Mikropartikelfraktion der Komponente d.)2 im Bereich von 1,5 : 1 bis 10 : 1, vorzugsweise 2 : 1 bis 5 : 1 liegt.

In einer besonders bevorzugten erfindungsgemäßen radikalisch härtbaren Zusammensetzung wird zumindest ein Teil der Mikropartikel der Komponente d.)2 durch organisch oberflächenmodifizierte Partikel, vorzugsweise silanisierte Partikel gebildet und/oder es wird zumindest ein Teil der Mikropartikel der Komponente d.)2 durch Glas-Partikel gebildet; vorzugsweise sind zumindest ein Teil der Mikropartikel der Komponente d.)2 organisch oberflächenmodifizierte Glas-Partikel, vorzugsweise silanisierte Glas-Partikel.

Bevorzugt zeichnet sich in diesen Fällen Komponente d.)2 durch eine bi- oder multimodale Partikelgrößenverteilung aus, insbesondere eine bi- oder multimodale Partikelgrößenverteilung mit den oben beschriebenen bevorzugten Merkmalen.

Neben den Komponenten d.)1 und d.)2 kann die radikalisch härtbare Zusammensetzung zu der Mischung von Füllstoffpartikeln zusätzlich weitere Füllstoffe als Komponente d.)3 umfassen.

So können z.B. verstärkend wirkende Füllstoff-Materialien, wie Glasfasern, Polyamid- oder Kohlenstofffasern eingesetzt werden. Eine erfindungsgemäße radikalisch härtbare Zusammensetzung kann auch feinteilige Splitter oder Perlpolymerisate enthalten, wobei die Perlpolymerisate Homo- oder Copolymere organischer härtbarer Monomerer sein können.

In einer besonders bevorzugten Ausführungsform für den Fall, dass Formteile für den medizinischen, insbesondere den dentalen Bereich durch ein generatives Fertigungsverfahren geplant sind, enthält eine erfindungsgemäße radikalisch härtbare Zusammensetzung einen röntgenopaken Füllstoff. Ganz besonders bevorzugt enthält die erfindungsgemäße Zusammensetzung nanoskaliges $YbF_3$ und/oder $BaSO_4$.

Qualitative und quantitative Charakterisierung der Füllstoffpartikel:

Die nachfolgend beschriebenen Schritte in der qualitativen und quantitativen Charakterisierung der Füllstoffpartikel (insbesondere von nanoskaligen Füllstoffpartikeln) sind dem Fachmann gut bekannt und in der Literatur umfassend beschrieben.

Harz-/Füllstofftrennung:

In einem ersten Schritt werden 1 g einer erfindungsgemäßen radikalisch härtbaren Zusammensetzung (nachfolgend auch Kompositmaterial genannt) in 10 ml Aceton resuspendiert und die erhaltene Suspension anschließend mit einer Zentrifuge für 10 min bei 5000 U/min zentrifugiert. Der Überstand (nachfolgend Harzphase genannt) wird in ein Sammelglas abdekantiert und der Rückstand in 5 ml Aceton aufgeschlämmt. Es wird erneut für 10 min bei 5000 U/min zentrifugiert, dekantiert und der Rückstand in 5 ml Aceton erneut aufgeschlämmt. Die Schritte Zentrifugieren, Dekantieren und Aufschlämmen werden noch zweimal unter identischen Bedingungen wiederholt. Die von den Harzphasen getrennte Gesamtmenge an Rückständen wird getrocknet und die Gesamtmenge an Harzphasen am Rotationsverdampfer von Aceton befreit.

Nach Durchführung des ersten Schrittes umfasst die getrocknete Gesamtmenge an Rückständen regelmäßig solche Füllstoffpartikel, die eine Partikelgröße von ca. 400 nm oder größer 400 nm besitzen (nachfolgend makroskopische Füllstoffpartikel genannt). Die von Aceton befreite Gesamtmenge an Harzphasen (nachfolgend Harzanteil genannt) umfasst neben polymerisierbaren Monomeren außerdem regelmäßig Füllstoffpartikel mit einer Partikelgröße von ca. 400 nm oder insbesondere kleiner 400 nm (nachfolgend nanoskalige Teilchen genannt). Dieses Verfahren stellt somit sicher, dass das Kompositmaterial durch die Zentrifugation in (i) einen Anteil makroskopischer Füllstoffpartikel, wobei speziell die Gläser im Größenordnungsbereich von größer 400 nm bis in den hohen Mikrometerbereich betroffen sind, und (ii) einen Harzanteil umfassend nanoskaligen Teilchen vollständig aufgetrennt wird.

Die mittlere Partikelgröße $d_{50}$ der erfindungsgemäß einzusetzenden makroskopischen Füllstoffpartikel der Füllstoffkomponente d.)2 einer erfindungsgemäßen Zusammensetzung wird mittels Lichtstreuung (Laserbeugung) bestimmt, vorzugsweise mit einem Partikelgrößenmessgerät Beckman Coulter LS 13320.

Die nanoskaligen Teilchen, die sich in dem Harzanteil befinden, können beispielsweise sowohl nicht-aggregierte

und/oder nicht agglomerierte, beispielsweise auch röntgenopake Partikel sein, beispielsweise $YbF_3$ oder $BaSO_4$ mit Partikelgrößen in einem Bereich von ca. 3 nm bis 200 nm, vorzugsweise von 5 nm bis 200 nm, besonders bevorzugt von 7 nm bis 100 nm und ganz besonders bevorzugt von 7 nm bis 70 nm aufweisen, als auch nicht-röntgenopake Kieselsäuren, die beispielsweise als pyrogene Kieselsäuren in Form von Aggregaten und/oder Agglomeraten mit einer Partikelgröße in einem Bereich von ca. 150 nm bis ca. 300 nm vorliegen oder auch Kieselsäuren, die nach dem Sol-Gel Verfahren (oder auch aus Wasserglas) synthetisiert werden und die ebenfalls nicht-aggregiert und/oder nicht-agglomeriert vorliegen und Partikelgrößen in einem Bereich von ca. 3 nm bis 200 nm, vorzugsweise von 5 nm bis 200 nm, besonders bevorzugt von 7 nm bis 100 nm und ganz besonders bevorzugt von 7 nm bis 70 nm aufweisen.

Der Gesamtmassenanteil anorganischer Partikel in dem Harzanteil wird durch Differenzwiegung nach Veraschung eines entsprechenden Harzanteils gravimetrisch bestimmt.

TEM in Kombination mit EELS:

In einem zweiten Schritt werden die Füllstoffpartikel in dem Harzanteil einer qualitativen und quantitativen Charakterisierung unterzogen. Hierzu wird TEM (Transmissionselektronenmikroskopie) in Verbindung mit EELS (Elektronenenergieverlustspektroskopie) eingesetzt.

Mittels TEM werden die Partikelgrößen der einzelnen Partikel sowie deren Anzahl ermittelt; eine Elementarbestimmung einzelner Partikel erfolgt mittels EELS.

Zur Durchführung der kombinierten TEM/EELS-Charakterisierung wird in einem ersten Schritt durch Verdünnung mit härtbarem Harz die Konzentration der nanoskaligen Teilchen im Harzanteil zunächst reduziert. Dadurch wird weitestgehend ausgeschlossen, dass eine "Überlagerung" von nanoskaligen Teilchen in den späteren Bildern beobachtet wird. Eine solche "Überlagerung" würde die Partikelcharakterisierung verfälschen. Eigene Untersuchungen haben gezeigt, dass die optimale Partikelkonzentration (d.h. der Volumenanteil der Füllstoffpartikel) für solche Untersuchungen bei 1 Vol.-% liegt, bezogen auf die Gesamtmasse der verdünnten Probe.

Aus den durch Verdünnung mit härtbarem Harz gewonnenen verdünnten Harzanteilen werden in einem zweiten Schritt durch Aushärtung Stäbchen hergestellt. Aus diesen Stäbchen werden anschließend mit einem Ultradiamantmesser (beispielsweise Ultramikrotom ULTRCAT UCT, LEICA, Wetzlar) mehrere 300 nm dünne Ultradünnschnitte angefertigt. Die Ultradünnschnitte werden zur Stabilisierung auf Kupfer-TEM-Grids transferiert. Es resultieren Dünnschnittpräparate. Diese Dünnschnittpräparate werden dann mit 120 kV Beschleunigungsspannung in einem TEM mittels Hellfeld-Abbildungen untersucht.

Eine TEM-Untersuchung an den vorstehend beschriebenen Dünnschichtpräparaten erlaubt es, nicht aggregierte und nicht agglomerierte nanoskalige Partikel von aggregierten und/oder agglomerierten Partikeln (z.B. Kieselsäuren, wie beispielsweise Aerosilen), zu unterscheiden (zur Identifizierung der chemischen Zusammensetzung siehe die nachfolgenden Ausführungen).

Sofern hochauflösende Abbildungen untersucht werden sollen, können Ultradünnschnitte mit Schichtdicken kleiner 100 nm hergestellt und untersucht werden.

In einem dritten Schritt werden die Füllstoffpartikel in den Ultradünnschnitten bzw. Dünnschnittpräparaten mittels EELS-Punktanalysen chemisch charakterisiert, so dass die chemische Zusammensetzung einzelner Partikel bekannt wird (zur Bestimmung der Oberflächenmodifikation von Partikeln siehe nachfolgende Punkte).

Die volumen- oder gewichtsbezogenen Anteile von (ggf. auch mehrerer) Partikelfraktionen werden in einem vierten Schritt wie folgt aus einer TEM Aufnahme ermittelt: Der im Mikroskop betrachtete Bildausschnitt einer TEM-Aufnahme stellt eine Fläche dar, deren Kantenlängen a und b mittels der Legende bestimmt werden. Multipliziert mit der Dicke c des Ultradünnschnittes ergibt sich ein Gesamtvolumen $V_{Gesamt}$ für den in der TEM betrachteten Bereich. Dieses Gesamtvolumen $V_{Gesamt}$ ist die Summe aus dem Harzvolumen $V_{Harz}$ und dem Volumen aller Partikel $V_{Partikel}$ innerhalb dieses Volumens (das Volumen aller Partikel umfasst ggf. mehrere Gruppen von Partikeln, z.B. sortiert nach verschiedenen Kriterien wie z.B. der Größe). Es gilt $V_{Gesamt} = a * b * c = V_{Harz} + V_{Partikel}$.

Das Volumen einzelner Partikel (und damit das Volumen aller Partikel in dem betrachteten Volumen) ist rechnerisch zugänglich über das Kugelvolumen der einzelnen Partikel. Hierzu wird in der TEM-Aufnahme der Durchmesser bzw. Radius eines entsprechenden Partikels bestimmt. Das daraus errechnete Kugelvolumen, multipliziert mit der Dichte des entsprechenden Materials, aus dem das Partikel besteht (Material identifizierbar mittels EELS), ergibt die Masse des Partikels. Das Harzvolumen, zugänglich aus dem Gesamtvolumen minus dem Partikelvolumen, multipliziert mit der Harzdichte, ergibt die Harzmasse. Die Harzdichte ergibt sich weitestgehend aus der Dichte des zur Verdünnung eingesetzten Harzes und ggf. der Dichte des verdünnten Harzanteils (letztere kann ggf. bei der Berechnung der Harzdichte vernachlässigt werden, wenn der Anteil des verdünnten Harzes vernachlässigbar ist). Der Anteil der Partikel (oder einer Gruppe von Partikeln) in Gewichtsprozent errechnet sich aus $m_P*100/(m_{Partikel}+m_{Harz})$, wobei $m_P$ die Masse der betrachteten Partikelfraktion in dem betrachteten Volumen, $m_{Partikel}$ die Masse aller Partikel im betrachteten Volumen und $m_{Harz}$ die Masse des Harzes in dem betrachteten Volumen bedeutet. Bei der abschließenden Berechnung des Gewichtsanteils der zu betrachtenden Partikelfraktion wird der Verdünnungsfaktor entsprechend berücksichtigt.

Bestimmung organischer Oberflächenmodifikationen:

Vorabbetrachtung:

Viele bekannte röntgenopake Füllstoffmaterialien (wie z.B. Ytterbiumfluorid oder Bariumsulfat) weisen den Nachteil auf, dass sie nur schwer in die Matrix (Harzmatrix) aus polymerisierbaren Monomeren (die sogenannte organische Harzphase) einzuarbeiten sind, weil sie keine hinreichenden chemischen Bindungen (Anbindungsmöglichkeiten) mit den hydrophoben Gruppen des Mediums eingehen. Glasartige Füllstoffe lassen sich beispielsweise mit Hilfe der Silanisierung über Si-OH Gruppen hervorragend in die Harzmatrix dentaler Kompositmaterialien einarbeiten. Bei Ytterbiumfluorid und Bariumsulfat sind solche Gruppen auf den Oberflächen nicht vorhanden; sie sind somit nicht silanisierbar und führen in einem gehärteten Dentalmaterial zu einer unzureichenden physikalischen und chemischen Resistenz (siehe hierzu WO 2005/011621 A1, Seite 2 unten).

Die in einem erfindungsgemäßen härtbaren Material eingesetzten röntgenopaken nanoskaligen Teilchen werden somit auf ihren Oberflächen keine Silane aufweisen. Vielmehr erfolgt die Verknüpfung über Stickstoff-, Sauerstoff-, Schwefel- und/oder Phosphoratomen (siehe hierzu wiederum WO 2005/011621 A1 sowie unsere Ausführungen weiter oben im Text).

Abtrennung polymerisierbarer Monomere von nanoskaligen Teilchen:

"Cross-Flow"-Verfahren:

Die Abtrennung polymerisierbarer Monomere von nanoskaligen Teilchen erfolgt beispielsweise in einem dem Fachmann bekannten "Cross-Flow"-Verfahren mittels Ultrafiltrationsmembranen.

Hierbei wird ein Harzanteil enthaltend nanoskalige Teilchen, polymerisierbare Monomere und ggf. ein geeignetes Verdünnungsmittel aus einem Behältnis mittels einer Pumpe in einen Kreislauf aus bestimmten Membranen gepumpt, wobei die polymerisierbaren Monomere die Poren der Membranen passieren und als Filtrat separiert werden, während die nanoskaligen Teilchen innerhalb des Kreislaufs (und damit im Behältnis) verbleiben.

Für diesen Trennungsgang ist beispielsweise das System "Vivaflow 50" von "Sartorius Stedim Biotech GmbH, Göttingen" geeignet. Pumpenantrieb (7554-95) und Pumpenkopf entstammen der Reihe "Masterflex L/S" von "Cole-Palmer Instrument Co.", Illinois, USA. Der Betrieb der Pumpe wird während der Filtration auf 2,5 bar eingestellt. Zwei Trennmembranen des Typs "50,000 MWCO (PES)" werden hintereinander geschaltet. Der MWCO (Molecular Weight Cut Off) gibt hierbei die Trenngrenze an, d.h. die Größe der Moleküle, die noch effizient die Membran passieren können. Dieser Wert wird in Dalton angegeben. Die erhaltenen Fraktionen werden anschließend wie unten beschrieben, untersucht.

Sedimentations-Feld-Fluss-Fraktionierung (SF3):

Besser noch als das "Cross-Flow"-Verfahren ist die Durchführung einer Sedimentations-Feld-Fluss-Fraktionierung (SF3). Dabei lassen sich insbesondere unterschiedliche Partikelfraktionen voneinander und zusätzlich vom Harzanteil trennen. Voraussetzung hierbei ist, dass sich die unterschiedlichen Partikelfraktionen ausreichend in Größe und/oder Dichte voneinander unterscheiden.

Entsprechende Geräte, die eine hierfür notwendige Trennsäule enthalten, sind bei der Firma Postnova Analytics GmbH, Landsberg, erhältlich. Das die Trennsäule enthaltende Modul trägt die Bezeichnung CF2000 Centrifugal FFF und wird durch die weiteren Module PN7140 (Eluent Organizer), PN1130 (Isocratic Pump), PN5300 (Autosampler), PN3621 MALS (21-Multi-Angle Light Scattering Detector) und PN8050 (Fraction Collector) ergänzt. In dieser Kombination erlaubt die Centrifugal FFF-Anlage nicht nur die analytische, sondern auch die präparative Trennung von Partikelfraktionen. Die erhaltenen Fraktionen werden anschließen wie unten beschrieben, untersucht.

Charakterisierung der Oberflächenmodifikation:

Eine wie oben hergestellte und anschließend von Lösungsmitteln befreite Probe, die nanoskalige Teilchen in Form eines Pulvers enthält, wird anschließend mittels spektroskopischer Verfahren untersucht (z.B. mittels 1H-NMR, 13C-NMR, 15N-NMR, 29Si-NMR und 31P-NMR sowie IR).

Signale, die sich nicht einem Silan, beispielsweise dem gamma-Methacryloxypropylsilylrest, zuordnen lassen, werden organischen Oberflächenmodifikationen zugeordnet, die nicht auf Silanen basieren, z.B. Oberflächenmodifikationen mittels organischer Verbindungen auf Oberflächen von Ytterbiumfluorid bzw. Bariumsulfatpartikeln.

Die Anteile organisch oberflächenmodifizierter Partikel bzw. nicht organisch oberflächenmodifizierter Partikel können regelmäßig auch durch Auswertung der Intensitäten entsprechender Schwingungsbanden im IR-Spektrum bestimmt werden. Hierzu werden üblicherweise Referenz-Schwingungsbanden (Referenzkurven) organisch oberflächenmodifizierter bzw. nicht organisch oberflächenmodifizierter Partikel mit den entsprechenden chemischen Zusammensetzungen herangezogen.

Charakterisierung mittels Bildanalyse und Ramanspektroskopie:

Dem Fachmann sind zusätzliche Verfahren bzw. Kopplungen von Verfahren bekannt, die eine qualitative und quantitative Charakterisierung der Füllstoffpartikel erlauben. Insoweit sei beispielsweise verwiesen auf den Artikel "Chemische Identität einzelner Partikel" von Deborah Huck-Jones und Renate Hessemann in "Nachrichten aus der Chemie", Volume 62, September 2014, Seiten 886 und 887. Die darin offenbarte Kombination von Bildanalyse und Ramanspektroskopie eignet sich regelmäßig auch zur Charakterisierung der Füllstoffpartikel im Rahmen der vorliegenden Erfindung. Dies gilt insbesondere für Proben, die nach der oben beschriebenen Harzfüllstofftrennung erhalten werden. Eine geeignete Bildanalyse ist z.B. auch die oben im Text beschriebene TEM-Analyse.

Bestandteil e.) - Initiatoren und/oder Katalysatoren für die radikalische Polymerisation

Eine erfindungsgemäße radikalisch härtbare Zusammensetzung ist vorzugsweise lichthärtbar und/oder chemisch härtbar. Bevorzugt ist eine erfindungsgemäße radikalisch härtbare dentale Zusammensetzung, wobei Bestandteil e.) einen oder mehrere Lichthärtungsinitiatoren und/oder einen oder mehrere Initiatoren zur chemischen Aushärtung umfasst oder aus diesen besteht.

Bevorzugte erfindungsgemäße radikalisch härtbare Zusammensetzungen sind lichthärtbar (photohärtbar) und umfassen Lichthärtungsinitiatoren. Beispiele für einen Lichthärtungsinitiator schließen Substanzen ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Bisacylphosphinoxide, Acylgermanium-Verbindungen, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1, oder in der DE 39 41 629 C2, welche im Wege der Verweisung Bestandteil der vorliegenden Anmeldung sind.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2, welche im Wege der Verweisung Bestandteil der vorliegenden Anmeldung sind.

Weitere geeignete Initiatoren sowie Initiatorkombinationen sind in der DE 601 16 142 beschrieben, welche im Wege der Verweisung Bestandteil der vorliegenden Anmeldung sind.

Das Absorptionsmaximum von Campherchinon (CQ) liegt bei ca. 470 nm und somit im Bereich des blauen Lichts. Campherchinon (CQ) zählt zu den $PI_2$-Initiatoren und wird regelmäßig zusammen mit einem Coinitiator eingesetzt.

Vorzugsweise enthält eine erfindungsgemäße Zusammensetzung die Kombination eines alpha-Diketons und eines aromatischen tertiären Amins, bevorzugt ist die Kombination von Campherchinon (CQ) und Ethyl-*p*-*N*,*N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in einer erfindungsgemäßen radikalisch härtbaren Zusammensetzung wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen, welche im Wege der Verweisung Bestandteil der vorliegenden Anmeldung sind.

Die in diesen Druckschriften angegebenen Phosphinoxide eigenen sich besonders allein oder in Kombination mit dem System "alpha-Diketon/Amin" als Photopolymerisationsinitiatorsystem in einer erfindungsgemäßen radikalisch härtbaren Zusammensetzung.

Die EP 1 905 415 beschreibt polymerisierbare Dentalzusammensetzungen mit Acylgermanium-Verbindungen als Initiatoren.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden, welche im Wege der Verweisung Bestandteil der vorliegenden Anmeldung sind.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben, welche im Wege der

Verweisung Bestandteil der vorliegenden Anmeldung sind.

Bestandteil f.) - weitere übliche Additive

Eine erfindungsgemäße radikalisch härtbare dentale Zusammensetzung umfasst in manchen Fällen ein oder mehrere weitere(s) Additiv(e).

Diese Additive können verschiedene Funktionen haben. Übliche Additive für den Einsatz in dentalen Werkstoffen sind dem Fachmann bekannt, je nach gewünschter Funktion wird er das oder die geeigneten Additive auswählen. Beispielhaft sind im Folgenden typische Additive und ihre Funktionen beschrieben.

Radikalisch lichthärtbare Zusammensetzungen, wie sie erfindungsgemäß bevorzugt sind, enthalten vorzugsweise einen oder mehrere Inhibitor(en), auch Stabilisator(en) genannt. Diese werden üblicherweise zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der lichthärtbaren dentalen Zusammensetzung. Gängige Inhibitoren sind Phenolderivate wie Hydrochinonmonomethylether (HQME) oder 2,6-Di-tert.-butyl-4-methylphenol (BHT). Weitere Inhibitoren wie tert.-Butylhydroxyanisol (BHA), 2,2 Diphenyl-1-picryl-hydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben, welche im Wege der Verweisung Bestandteil der vorliegenden Anmeldung sind. Alternative Inhibitoren sind in der DE 101 19 831 A1 oder in der EP 1 563 821 A1 angegeben, welche im Wege der Verweisung Bestandteil der vorliegenden Anmeldung sind.

Eine erfindungsgemäß bevorzugte radikalisch härtbare Zusammensetzung umfasst somit als Additiv ein oder mehrere Polymerisationsinhibitoren zur Erhöhung der Lagerstabilität der Zusammensetzung, vorzugsweise ausgewählt aus der Gruppe bestehend aus Hydrochinonmonomethylether (HQME), Phenolen, dabei vorzugsweise 2,6-Di-tert.-butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten.

UV-Absorber, die beispielsweise durch ihre konjugierten Doppelbindungssysteme und aromatischen Ringe befähigt sind, UV Strahlung zu absorbieren, sind in manchen Fällen Bestandteil einer erfindungsgemäßen radikalisch härtbaren dentalen Zusammensetzung. Als Absorber eignen sich insbesondere Benzotriazole, Triazine, Benzophenone, Cyanoacrylate, Salicylsäurederivate, Hindered Amine Light Stabilizers (HALS) sowie Mischungen davon. Beispiele an UV-Absorbern sind 2-Hydroxy-4-methoxybenzophenon, Salicylsäurephenylester 3-(2'-Hydroxy-5'-methylphenyl)benzotriazol oder Diethyl-2,5-dihydroxyterephthalat. Weitere Absorber sind dem Experten aus der WO 2013/153183 A2, welche im Wege der Verweisung Bestandteil der vorliegenden Anmeldung sind, bekannt.

Weitere optionale Additive sind Aromastoffe, Pigmente, organische Farbstoffe, organische Polymere und Oligomere, vorzugsweise Weichmacher, Mikrobizide, vorzugsweise Bakterizide, grenzflächenaktive Substanzen, vorzugsweise Tenside, Konservierungsmittel oder Molekulargewichtsregler.

In einer bevorzugten erfindungsgemäßen Verwendung umfasst die Zusammensetzung die Komponente

a.) zu 10 - 70 Gew.-%, vorzugsweise zu 10 - 35 Gew.-%,

b.) zu 2 - 40 Gew.-%, vorzugsweise zu 2 - 25 Gew.-%,

c.) zu 0 - 30 Gew.-%, vorzugsweise zu 0 - 20 Gew.-%,

d.) zu 0 - 85 Gew.-%, vorzugsweise zu 40 - 78 Gew.-%,

e.) zu 0,001 - 5 Gew.-%, vorzugsweise zu 0,1 - 2 Gew.-% und

f.) zu 0,001 - 20 Gew.-%, vorzugsweise zu 0,001 - 10 Gew.-%,

wobei die jeweiligen Gewichtsprozente auf die Gesamtmasse der Zusammensetzung bezogen sind.

In besonders bevorzugten erfindungsgemäßen Verwendungen enthält die radikalisch härtbare Zusammensetzung keinen Bestandteil c.).

In besonders bevorzugten erfindungsgemäßen Verwendungen umfasst eine radikalisch härtbare Zusammensetzung das 1,3-Bis(3-methacryloxypropyl)tetramethyldisiloxan als Bestandteil b.).

In einer ganz besonders bevorzugten erfindungsgemäßen Verwendung enthält eine radikalisch härtbare Zusammensetzung keinen Bestandteil c.) und das 1,3-Bis(3-methacryloxypropyl)tetramethyldisiloxan als Bestandteil b.).

In einer bevorzugten erfindungsgemäßen Verwendung umfasst das generative Fertigungsverfahren die Stereolithographie, das digital light processing, die Polyjettechnologie, das 3D plotting, die Galvanometer type scanning

method, die Mikrostereolithographie, das Multi-Jet Modelling, das selektive Lasersintern, das 3D-Printing, das Fused Deposition Modelling, das Laminated Object Manufacturing oder das Film Transfer Imaging.

In einer besonders bevorzugten erfindungsgemäßen Verwendung umfasst das generative Fertigungsverfahren die Stereolithographie, das digital light processing und die Polyjettechnologie.

In einer ganz besonders bevorzugten erfindungsgemäßen Verwendung umfasst das generative Fertigungsverfahren die Stereolithographie.

In einer besonders bevorzugten erfindungsgemäßen Verwendung wird ein dentales Formteil hergestellt.

In einer ganz besonders bevorzugten erfindungsgemäßen Verwendung ist das dentale Formteil ein Inlay, ein Onlay, ein Veneer, eine Krone, eine Brücke, ein künstlicher Zahn, ein Zahnfertigteil, ein Gerüst, ein Provisorium, eine Teil- oder Vollprothese sowie ein KFO-Produkt.

Die vorliegende Erfindung betrifft außerdem ein Verfahren zur Herstellung eines Formteils, vorzugsweise eines dentalen Formteils, bei dem eine radikalisch härtbare Zusammensetzung wie vorstehend definiert, schichtweise durch Einbringen von Strahlung unter Ausbildung eines dreidimensionalen Körpers gehärtet wird:

Beispiele

Eingesetzte Substanzen:

| | |
|---|---|
| Polysiloxan I: | Methacryl-POSS (MA0735, Hybrid Plastics Inc.) |
| Polysiloxan II: | Kondensationsprodukt aus 3-Methacryloxypropyldimethoxymethylsilan |
| Polysiloxan III: | Kondensationsprodukt aus 3-Methacryloxypropyltrimethoxysilan |
| Polysiloxan IV: | Kondensationsprodukt aus 3-[(2-Hydroxy-3-methacryloxy)propoxy]propyldimethoxymethylsilan |
| Disiloxan I: | 1,3-Bis(3-methacryloxypropyl)tetramethyldisiloxan |
| Disiloxan II: | 1,3-Bis[3-[(2-hydroxy-3-methacryloxy)propoxy]propyl]-tetramethyldisiloxan |
| Bis-GMA: | 2,2-Bis[4-(2-hydroxy-3-methacryloxypropoxy)phenyl]propan |
| UDMA | 7,7,9-Trimethyl-4,13-dioxo-5,12-diazahexadecan-1,16-dioxydimethacrylat |
| DDM | Dodecandioldimethacrylat |
| CQ: | DL-Kampferchinon |
| DABE: | 4-Dimethylaminobenzoesäureethylester |
| TPO: | 2,4,6-Trimethylbenzoyldiphenylphosphinoxid |
| Tinuvin 571 | 2-(2H-Benzotriazol-2-yl)-6-dodecyl-4-methyl-phenol |
| BHT: | 2,6-Di(tert.-butyl)hydroxytoluol |

Synthese der Polysiloxane

Synthese von Polysiloxan II

100 g (0,42 mol) 3-Methacryloxypropyldimethoxymethylsilan werden in 400 ml Essigester gelöst. Es werden 10 ml 1N HCl-Lösung zugetropft und für 72 h bei 30°C gerührt. Es wird mit 2N NaOH-Lösung ausgeschüttelt, mit Wasser gewaschen und die organische Phase über Magnesiumsulfat getrocknet. Nach Zusatz von BHT wird zunächst bei 40 °C abrotiert und anschließend werden Lösungsmittelreste (z.B. Wasser- und Alkoholreste) unter Vakuum mittels einer Ölpumpe abgezogen, um die Alkohol- und Wasserreste zu entfernen. Es resultiert ein flüssiges Harz mit einer Viskosität von 3 Pa*s bei 25 °C.
$n_D^{20}$ = 1,466

Synthese von Polysiloxan III

100 g (0,40 mol) 3-Methacryloxypropyltrimethoxysilan werden in 400 ml Essigester gelöst. Es werden 15 ml 1N HCl-Lösung zugetropft und für 72 h bei 30°C gerührt. Es wird mit 2N NaOH-Lösung ausgeschüttelt, mit Wasser gewaschen und die organische Phase über Magnesiumsulfat getrocknet. Nach Zusatz von BHT wird zunächst bei 40 °C abrotiert und anschließend werden Lösungsmittelreste (z.B. Wasser- und Alkoholreste) unter Vakuum mittels einer Ölpumpe abgezogen, um die Alkohol- und Wasserreste zu entfernen. Es resultiert ein flüssiges Harz mit einer Viskosität von 20 Pa*s bei 25 °C.
$n_D^{20}$ = 1,479

Synthese von Polysiloxan IV

a) Synthese einer monomeren Silaneinheit (vgl. EP 1 685 182 B1, Bsp. 3):

Zur Vorlage von 100 g (0,402 mol) 3-Glycidyloxypropyldiethoxymethylsilan werden unter trockener Atmosphäre ein Additionskatalysator, BHT als Stabilisator und anschließend 38,05 g (0,442 mol) Methacrylsäure zugetropft und bei ca. 80 °C gerührt (ca. 24 h). Die Umsetzung wird über die Abnahme der Carbonsäurekonzentration mittels Säuretitration sowie dem Epoxidumsatz mittels Ramanspektroskopie/Epoxidtitration verfolgt. Die für die Epoxidgruppe charakteristische Bande wird im Ramanspektrum bei 1256 $cm^{-1}$ detektiert. Der Epoxid- bzw. Carbonsäureumsatz liegt bei 99% bzw. 88% (Folge des Carbonsäureüberschusses).

b) Hydrolyse bzw. Kondensation der monomeren Silaneinheit zu einer Polysiloxanverbindung (vgl. EP 1 685 182 B1, Bsp. 6):

Nach Zugabe von Essigester (1000 ml/mol monomerer Silaneinheit) und Wasser zur Hydrolyse mit HCl als Katalysator zur synthetisierten monomeren Silaneinheit wird bei 30 °C gerührt. Der Verlauf der Hydrolyse wird durch Wassertitration verfolgt. Die Aufarbeitung erfolgt nach mehrtägigem Rühren bei 30°C durch mehrmaliges Ausschütteln mit wässriger NaOH und anschließendem Ausschütteln mit Wasser und Filtration über einen hydrophobierten Filter. Nach Zusatz von BHT wird zunächst bei 40 °C abrotiert und anschließend werden Lösungsmittelreste (z.B. Wasser- und Alkoholreste) unter Vakuum mittels einer Ölpumpe abgezogen, um die Alkohol- und Wasserreste zu entfernen. Es resultiert ein flüssiges Harz mit einer Viskosität von 4,5 Pa*s bei 25 °C.
$n_D^{20}$ = 1,483

Synthese der Disiloxane

Synthese von Disiloxan II

Zur Vorlage von 100 g (0,28 mol) 1,3-Bis(glycidoxypropyl)disiloxan werden unter trockener Atmosphäre ein Additionskatalysator, BHT als Stabilisator und anschließend 26,10 g (0,30 mol) Methacrylsäure zugetropft und bei ca. 80 °C gerührt (ca. 24 h). Die Umsetzung wird über die Abnahme der Carbonsäurekonzentration mittels Säuretitration sowie dem Epoxidumsatz mittels Ramanspektroskopie/Epoxidtitration verfolgt. Die für die Epoxidgruppe charakteristische Bande wird im Ramanspektrum bei 1256 $cm^{-1}$ detektiert. Der Epoxid- bzw. Carbonsäureumsatz liegt bei 99% bzw. 88% (Folge des Carbonsäureüberschusses). Es werden 300 ml Essigester zugegeben. Anschließend wird mit 2N NaOH-Lösung ausgeschüttelt, mit Wasser gewaschen und die organische Phase über Magnesiumsulfat getrocknet. Nach Zusatz von BHT wird das Lösungsmittel unter Vakuum entfernt. Es resultiert ein dünnflüssiges Harz mit einer Viskosität von 0,3 Pa*s bei 25 °C.
$n_D^{20}$ = 1,466

Lichthärtende Polysiloxan/Disiloxan-Harze (Beispiele 1-4)

CQ und DABE wurden in den jeweiligen Monomeren (Polysiloxan und Disiloxan) gelöst. Die Lösungen wurden bei -0,9 bar Vakuum entlüftet. Es wurde die Biegefestigkeit (BF) der einzelnen Harze bestimmt und aus diesen Messungen der Elastizitätsmodul (E-Modul) berechnet.

Überraschenderweise wird dabei für die Mischungen aus Polysiloxan und Disiloxan ein höherer E-Modul gefunden als für die jeweiligen beiden Einzelkomponenten. Das Maximum für den E-Modul wurde bei einem Verhältnis von Polysiloxan zu Disiloxan von etwa 2:1 gefunden (siehe Abbildung 1). Ein höherer E-Modul bedeutet, dass das gehärtete Material seiner Verformung einen größeren Widerstand entgegensetzen kann und so den stetigen Kaubelastungen besser widerstehen kann.

Beispiel 1:

Tabelle 1: Zusammensetzung und Eigenschaften Poly-/Disiloxan-Harze 1

| | 1-A (Vergleich) | 1-B | 1-C | 1-D | 1-E (Vergleich) |
|---|---|---|---|---|---|
| Polysiloxan I | 99,25 | 82,71 | 66,17 | 49,625 | 0,00 |

(fortgesetzt)

| | 1-A (Vergleich) | 1-B | 1-C | 1-D | 1-E (Vergleich) |
|---|---|---|---|---|---|
| Disiloxan I | 0,00 | 16,54 | 33,08 | 49,625 | 99,25 |
| CQ | 0,30 | 0,30 | 0,30 | 0,30 | 0,30 |
| DABE | 0,45 | 0,45 | 0,45 | 0,45 | 0,45 |
| BF | 9,9 MPa | 20,8 MPa | 25,2 MPa | 28,2 MPa | 36,3 MPa |
| E-Modul | 560 MPa | 960 MPa | 1040 MPa | 1020 MPa | 880 MPa |

Beispiel 2:

Tabelle 2: Zusammensetzung und Eigenschaften Poly-/Disiloxan-Harze 2

| | 2-A (Vergleich) | 2-B | 2-C | 2-D | 2-E (Vergleich) |
|---|---|---|---|---|---|
| Polysiloxan II | 99,25 | 82,71 | 66,17 | 49,625 | 0,00 |
| Disiloxan I | 0,00 | 16,54 | 33,08 | 49,625 | 99,25 |
| CQ | 0,30 | 0,30 | 0,30 | 0,30 | 0,30 |
| DABE | 0,45 | 0,45 | 0,45 | 0,45 | 0,45 |
| BF | 13,0 MPa | 22,3 MPa | 23,0 MPa | 27,8 MPa | 36,3 MPa |
| E-Modul | 920 MPa | 1140 MPa | 1200 MPa | 1130 MPa | 880 MPa |

Beispiel 3:

Tabelle 3: Zusammensetzung und Eigenschaften Poly-/Disiloxan-Harze 3

| | 3-A (Vergleich) | 3-B | 3-C | 3-D | 3-E (Vergleich) |
|---|---|---|---|---|---|
| Polysiloxan III | 99,25 | 82,71 | 66,17 | 49,625 | 0,00 |
| Disiloxan I | 0,00 | 16,54 | 33,08 | 49,625 | 99,25 |
| CQ | 0,30 | 0,30 | 0,30 | 0,30 | 0,30 |
| DABE | 0,45 | 0,45 | 0,45 | 0,45 | 0,45 |
| BF | 19,6 MPa | 31,3 MPa | 32,0 MPa | 31,7 MPa | 36,3 MPa |
| E-Modul | 1050 MPa | 1360 MPa | 1380 MPa | 1250 MPa | 880 MPa |

Beispiel 4:

Tabelle 4: Zusammensetzung und Eigenschaften Poly-/Disiloxan-Harze 4

| | 4-A (Vergleich) | 4-B | 4-C | 4-D | 4-E (Vergleich) |
|---|---|---|---|---|---|
| Polysiloxan IV | 99,25 | 82,71 | 66,17 | 49,625 | 0,00 |
| Disiloxan II | 0,00 | 16,54 | 33,08 | 49,625 | 99,25 |
| CQ | 0,30 | 0,30 | 0,30 | 0,30 | 0,30 |
| DABE | 0,45 | 0,45 | 0,45 | 0,45 | 0,45 |

(fortgesetzt)

| | 4-A (Vergleich) | 4-B | 4-C | 4-D | 4-E (Vergleich) |
|---|---|---|---|---|---|
| BF | 25,7MPa | 36,3 MPa | 38,1 MPa | 34,5 MPa | 28,4 MPa |
| E-Modul | 1260 MPa | 1530 MPa | 1550 MPa | 1470 MPa | 1090 MPa |

Herstellung und Eigenschaften der Komposit-Pasten (Beispiel 5)

Für die Herstellung der Harzmischungen 5-A bis D wurden TPO, BHT und Tinuvin 571 jeweils in den angegebenen Monomeren gelöst und die Lösungen wurden bei -0,9 bar Vakuum entlüftet.

Für die Herstellung Komposit-Pasten 5-E bis L wurden die Bestandteile jeweils eingewogen an einem SpeedMixer™ DAC 600.1 VAC-P (Hauschild & Co KG, Hamm, Deutschland) homogenisiert, an einem Dreiwalzenstuhl (Exakt, Norderstedt, Deutschland) gewalzt und anschließend am SpeedMixer™ DAC 600.1 VAC-P bei -0,9 bar Vakuum entlüftet.

Tabelle 5: Zusammensetzungen der Beispiele 5-A bis F

| | 5-A | 5-B (Vergleich) | 5-C | 5-D (Vergleich) | 5-E | 5-F (Vergleich) |
|---|---|---|---|---|---|---|
| Polysiloxan IV | 86,76 | 0,00 | 84,90 | 0,00 | 30,05 | 0,00 |
| Disiloxan I | 11,89 | 0,00 | 11,58 | 0,00 | 18,00 | 0,00 |
| Bis-GMA | 0,00 | 40,00 | 0,00 | 39,12 | 0,00 | 15,24 |
| UDMA | 0,00 | 38,65 | 0,00 | 37,79 | 0,00 | 14,72 |
| DDM | 0,00 | 20,00 | 0,00 | 19,57 | 0,00 | 18,09 |
| Bariumsilikat–Glas (1,5 µm) silanisiert | 0,00 | 0,00 | 0,00 | 0,00 | 0,00 | 0,00 |
| Bariumsilikat–Glas (0,7 µm) silanisiert | 0,00 | 0,00 | 0,00 | 0,00 | 24,66 | 24,66 |
| nanosklaliges $SiO_2$ (40 nm) silanisiert | 0,00 | 0,00 | 0,00 | 0,00 | 24,66 | 24,66 |
| TPO | 1,30 | 1,30 | 1,30 | 1,30 | 0,65 | 0,65 |
| Tinuvin 571 | 0,00 | 0,00 | 2,17 | 2,17 | 1,93 | 1,93 |
| BHT | 0,05 | 0,05 | 0,05 | 0,05 | 0,05 | 0,05 |

Tabelle 5 (Fortsetzung): Zusammensetzungen der Beispiele 5-G bis L

| | 5-G | 5-H | 5-I | 5-J | 5-K | 5-L (Vergleich) |
|---|---|---|---|---|---|---|
| Polysiloxan IV | 24,03 | 32,05 | 22,05 | 16,16 | 28,05 | 0,00 |
| Disiloxan I | 14,40 | 16,00 | 14,00 | 10,27 | 8,00 | 0,00 |
| Bis-GMA | 3,05 | 0,00 | 0,00 | 3,05 | 0,00 | 11,43 |
| UDMA | 2,95 | 0,00 | 0,00 | 2,95 | 0,00 | 11,03 |
| DDM | 3,62 | 0,00 | 0,00 | 3,62 | 0,00 | 13,59 |
| Bariumsilikat–Glas (1,5 µm) silanisiert | 0,00 | 16,44 | 32,55 | 32,55 | 32,55 | 32,55 |
| Bariumsilikat–Glas (0,7 µm) silanisiert | 24,66 | 8,22 | 4,11 | 4,11 | 16,44 | 4,11 |
| nanosklaliges $SiO_2$ (40 nm) silanisiert | 24,66 | 24,66 | 24,66 | 24,66 | 12,33 | 24,66 |
| TPO | 0,65 | 0,65 | 0,65 | 0,65 | 0,65 | 0,65 |
| Tinuvin 571 | 1,93 | 1,93 | 1,93 | 1,93 | 1,93 | 1,93 |
| BHT | 0,05 | 0,05 | 0,05 | 0,05 | 0,05 | 0,05 |

Tabelle 6: Eigenschaften der Zusammensetzungen der Beispiele 5-A bis F

| | 5-A | 5-B (Vergleich) | 5-C | 5-D (Vergleich) | 5-E | 5-F (Vergleich) |
|---|---|---|---|---|---|---|
| Abweichung x | +8,98% | +39,33% | +1,93% | +15,65% | +4,87% | +36,93% |
| Abweichung y | +11,49% | +101,83% | +3,72% | +17,92% | +6,69% | +39,31% |
| Viskosität | 3 Pa*s | 3 Pa*s | 3 Pa*s | 3 Pa*s | 5 Pa*s | 10 Pa*s |
| Schrumpfung | n.b. | n.b. | n.b. | n.b. | 3,8% | 4,8% |
| BF | n.b. | n.b. | n.b. | n.b. | 55 MPa | 60 MPa |
| E-Modul | n.b. | n.b. | n.b. | n.b. | 1,5 GPa | 1,6 GPa |

Tabelle 6 (Fortsetzung): Eigenschaften der Zusammensetzungen der Beispiele 5-G bis L

| | 5-G | 5-H | 5-I | 5-J | 5-K | 5-L (Vergleich) |
|---|---|---|---|---|---|---|
| Abweichung x | +11,72% | +8,59% | +13,58% | +20,34% | +21,54% | +41,85% |
| Abweichung y | +12,45% | +9,86% | +14,32% | +22,58% | +22,21% | +43,66% |
| Viskosität | 8 Pa*s | 7 Pa*s | 18 Pa*s | 22 Pa*s | 19 Pa*s | 28 Pa*s |
| Schrumpfung | 4,2% | 3,7% | 3,6% | 3,9% | 3,5% | 4,6% |
| BF | 55 MPa | 55 MPa | 85 MPa | 95 MPa | 85 MPa | 100 MPa |
| E-Modul | 1,5 GPa | 1,5 GPa | 5,0 GPa | 5,2 GPa | 5,5 GPa | 5,3 GPa |

Durch die Verwendung von Polysiloxan/Disiloxan-Harzen kann im Vergleich zu klassischen Bis-GMA-basierten Harzen die Schrumpfung gesenkt und die Genauigkeit (geringere Abweichung der Dimensionen) der Formköperherstellung deutlich verbessert werden. So ist für die reinen Harzmischungen die Abweichung des Polysiloxan/Disiloxan-Harzes 5-A (+9% bzw. +11%) deutlich besser als die des klassischen Bis-GMA-basierten Harzes 5-B (+39% bzw. +102%). Durch Verwendung von Tinuvin 571 lässt sich die Abweichung für beide Harze (5-C und D) deutlich senken. Das Polysiloxan/Disiloxan-Harz 5-C schneidet auch hier deutlich besser ab und das Bis-GMA-basierte Harz 5-D erreicht trotz des Absorbers immer noch nicht die Genauigkeit des Polysiloxan/Disiloxan-Harzes 5-A ohne Absorber. Mit steigendem Füllstoffgrad nimmt die Genauigkeit ab bzw. die Abweichung zu. Auch hier scheiden die Polysiloxan/Disiloxan-Harze (5-E bzw. 5-I) im Vergleich zu den Bis-GMA-basierten Harzen (5-F bzw. 5-L) deutlich besser ab und ihre Schrumpfung ist geringer.

Bestimmung der Eigenschaften:

Biegefestigkeit (BF): Die Biegefestigkeiten für die Beispiele 1-4 wurden entsprechend ISO 4049 bestimmt. Die Harze wurden mit einer Celalux 2-Lampe (VOCO GmbH) abschnittsweise für 40 Sekunden lichtgehärtet. Die Bestimmung der Biegefestigkeit erfolgte bei einer Vorschubgeschwindigkeit von 0,75 mm/min an einer Zwick-Universalprüfmaschine (Zwick GmbH & Co. KG, Ulm). Für die Bestimmung der Biegefestigkeiten der Beispiele 5 wurden jeweils 10 Proben stereolithographisch mit einer Wellenlänge von 405 nm (Form 1, Formlabs) hergestellt. Die Reinigung der BF-Proben erfolgte durch Spülen mit Isopropanol und einer anschließenden Trocknung mittels Druckluft, so dass keine Lösungsmittelrückstände mehr auf der Oberfläche zurückbleiben. Die Nachhärtung wurde mit einem Dentacolor XS Polymerisationsgerät (Kulzer) für 10 min durchgeführt. Alle Proben wurden für die Bestimmung der Biegefestigkeit mit einem SiC-Schleifpier (P2500 und P4000) nachgeschliffen. Die Bestimmung der Biegefestigkeit erfolgte analog der Beispiele

1-4 bei einer Vorschubgeschwindigkeit von 0,75 mm/min an einer Zwick-Universalprüfmaschine (Zwick GmbH & Co. KG, Ulm).

Elastizitätsmodul (E-Modul): Der Elastizitätsmodul wird aus der Steigung im elastischen Bereich der Kraft-Weg-Kurven der Biegefestigkeitsmessungen ermittelt.

Abweichung: Für die Bestimmung der Abweichung wurden die Prüfkörper (n=10) der Biegefestigkeitsmessungen vor dem Beschleifen verwendet. Mit einer Mikrometerschraube wurden pro Prüfkörper drei Werte in X-Richtung und drei Werte in Y-Richtung auf ±0,001 mm genau bestimmt. Aus diesen Werten und den Vorgabedaten von 2,000 mm wurde die Abweichung berechnet.

Schrumpfung: Die Schrumpfung wurde nach der Bonded-Disk-Methode von Watts et al. bestimmt (Watts DC, Cash AJ. Determination of polymerization kinetics in visible-light cured materials: Methods development. Dent Mater 1991; 7:281-287). Abweichend wurde mit einer Celalux 2-Lampe (VOCO GmbH) für 40 Sekunden pro Messung belichtet.

Viskosität: Die Bestimmung der Viskosität erfolgt mittels eines Rheometers (Physica MCR 301) der Firma Anton Paar (Graz, Österreich). Die Messung erfolgt bei 23 °C im Rotationsversuch mit Platte/Platte-Anordnung (Durchmesser 25 mm, Spaltabstand 1 mm) in einem Scherratenbereich von $10^{-2}$ bis 10 $s^{-1}$. Es werden pro Messung 16 Messwerte in einem Intervall von 30 Sekunden je Scherrate aufgenommen. In den Tabellen sind jeweils die Viskositäten für die Scherrate 10 $s^{-1}$ angegeben.

Für die generative Herstellung dentaler Formteile sind weitere Geräte sowie Technologien geeignet. Beispielsweise können Geräte wie Perfactory 4 DDP (EnisionTec GmbH), DigitalWax D (DWS Systems), iPro/ProJet/ProX (3D Systems), D30/ D30 L (Rapid Shape GmbH) und Freeform (Asiga Global Helpdesk) verwendet werden.

## Patentansprüche

**1.** Verwendung einer radikalisch härtbaren Zusammensetzung enthaltend

a.) mit radikalisch polymerisierbaren Gruppen substituierte kettenförmige und/oder zyklische und/oder in Käfigform vorliegende Polysiloxane mit mindestens 3 Siliziumatomen und/oder deren Mischformen, wobei die Polysiloxane a.) durch Hydrolyse oder Teilhydrolyse und anschließende Kondensation oder Cokondensation von ein, zwei, drei oder mehr Verbindungen $R^1{}_aR^2{}_bSiX_c$ mit
X: Halogen oder Alkoxy
$R^2$: Alkyl, Alkenyl, Aryl, Alkylaryl, Arylalkyl, wobei unterschiedliche $R^2$ gleich oder verschieden sein können,
$R^1$: YZ,
Z: radikalisch polymerisierbare Gruppe, ausgewählt aus den Strukturelementen $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ und $-NH-(C=O)-C(CH_3)=CH_2$,
wobei unterschiedliche Z gleich oder verschieden sein können,

a: 1 oder 2,
b: 0 oder 1,
c: 2 oder 3,

$$a + b + c = 4,$$

Y: ein Verbindungselement, das das Siliziumatom mit der radikalisch polymerisierbaren Gruppe verknüpft und aus einer Alkylengruppe besteht,
wobei die Alkylenguppe eine unsubstituierte, durchgängige, geradkettige oder verzweigte Kohlenwasserstoffkette ist oder
wobei die Alkylengruppe eine unsubstituierte, durch eine Urethangruppe, Harnstoffgruppe, Estergruppe, Thiourethangruppe oder Amidgruppe unterbrochene Kohlenwasserstoffgruppe ist oder
wobei die Alkylengruppe eine durch eine Hydroxylgruppe substituierte Kohlenwasserstoffgruppe ist oder diese Hydroxylgruppe verestert oder verethert ist oder
wobei die Alkylengruppe eine durch ein Sauerstoffatom und/oder Stickstoffatom und/oder Schwefelatom und/oder Estergruppen und/oder Thioestergruppen unterbrochene und durch eine Hydroxylgruppe substituierte Kohlenwasserstoffgruppe ist oder diese Hydroxylgruppe verestert oder verethert ist und
wobei unterschiedliche Y gleich oder verschieden sein können und
wobei Y 20 oder weniger Kohlenstoffatome enthält und
wobei YZ so gewählt wird, dass Z immer möglichst viele Atome aufweist,

erhalten werden,

b.) mit radikalisch polymerisierbaren Gruppen substituierte Disiloxane der folgenden Struktur

$$R^1{}_aR^2{}_{(3-a)}Si\text{-}O\text{-}SiR^2{}_{(3-b)}R^1{}_b$$

mit

$R^2$: Alkyl, Alkenyl, Aryl, Alkylaryl, Arylalkyl, wobei unterschiedliche $R^2$ gleich oder verschieden sein können,
$R^1$: YZ,
Z: radikalisch polymerisierbare Gruppe, ausgewählt aus den Strukturelementen $-O-(C{=}O)-CH{=}CH_2$, $-O-(C{=}O)-C(CH_3){=}CH_2$, $-(C{=}O)-CH{=}CH_2$, $-(C{=}O)-C(CH_3){=}CH_2$, $-CH{=}CH_2$, $-C(CH_3){=}CH_2$, $-NH-(C{=}O)-CH{=}CH_2$ und $-NH-(C{=}O)-C(CH_3){=}CH_2$,
wobei unterschiedliche Z gleich oder verschieden sein können,

a: 1 oder 2,
b: 1 oder 2,

Y: ein Verbindungselement, das das Siliziumatom mit der radikalisch polymerisierbaren Gruppe verknüpft und aus einer Alkylengruppe besteht,
wobei die Alkylenguppe eine unsubstituierte, durchgängige, geradkettige oder verzweigte Kohlenwasserstoffkette ist oder
wobei die Alkylengruppe eine unsubstituierte, durch eine Urethangruppe, Harnstoffgruppe, Estergruppe, Thiourethangruppe oder Amidgruppe unterbrochene Kohlenwasserstoffgruppe ist oder
wobei die Alkylengruppe eine durch eine Hydroxylgruppe substituierte Kohlenwasserstoffgruppe ist oder diese Hydroxylgruppe verestert oder verethert ist oder
wobei die Alkylengruppe eine durch ein Sauerstoffatom und/oder Stickstoffatom und/oder Schwefelatom und/oder Estergruppen und/oder Thioestergruppen unterbrochene und durch eine Hydroxylgruppe substituierte Kohlenwasserstoffgruppe ist oder diese Hydroxylgruppe verestert oder verethert ist und
wobei unterschiedliche Y gleich oder verschieden sein können und
wobei Y 20 oder weniger Kohlenstoffatome enthält und
wobei YZ so gewählt wird, dass Z immer möglichst viele Atome aufweist,

c.) optional ein, zwei, drei oder mehrere radikalisch härtbare Monomere ohne Siliziumatom,
d.) 85 Gewichtsprozent oder weniger Füllstoffe bezogen auf das Gesamtgewicht der radikalisch härtbaren dentalen Zusammensetzung,
e.) Initiatoren und/oder Katalysatoren für die radikalische Polymerisation und
f.) weitere übliche Additive

in einem generativen Fertigungsverfahren zur Herstellung eines dentalen Formteils, wobei das dentale Formteil ein Inlay, ein Onlay, ein Veneer, eine Krone, eine Brücke, ein künstlicher Zahn, ein Zahnfertigteil, ein Gerüst, ein Provisorium, eine Teil- oder Vollprothese sowie ein KFO-Produkt ist.

**2.** Verwendung einer radikalisch härtbaren Zusammensetzung nach Anspruch 1, wobei die Zusammensetzung die Komponente

a.) zu 10 - 70 Gew.-%, vorzugsweise 10 - 35 Gew.-%,
b.) zu 2 - 40 Gew.-%, vorzugsweise 2 - 25 Gew.-%,
c.) zu 0 - 30 Gew.-%, vorzugsweise 0 - 20 Gew.-%,
d.) zu 0 - 85 Gew.-%, vorzugsweise 40 - 78 Gew.-%,
e.) zu 0,001 - 5 Gew.-%, vorzugsweise 0,1 - 2 Gew.-% und
f.) zu 0,001 - 20 Gew.-%, vorzugsweise 0,001 - 10 Gew.-%

enthält und wobei die jeweiligen Gewichtsprozente auf die Gesamtmasse der Zusammensetzung bezogen sind.

**3.** Verwendung einer radikalisch härtbaren Zusammensetzung nach einem der Ansprüche 1, oder 2, wobei die Zusammensetzung als Bestandteil b.) das 1,3-Bis (3-methacryloxypropyl)tetramethyldisiloxan) enthält.

**4.** Verwendung einer radikalisch härtbaren Zusammensetzung nach einem der Ansprüche 1, 2, oder 3, wobei die

Zusammensetzung keinen Bestandteil c.) enthält.

5. Verwendung einer radikalisch härtbaren Zusammensetzung nach einem der vorangehenden Ansprüche, enthaltend eine Mischung aus Füllstoffen d. umfassend:

d.)1. organisch oberflächenmodifizierte anorganische Nanopartikel mit einer mittleren Teilchengröße kleiner 200 nm,
d.)2. anorganische Mikropartikel mit einer mittleren Teilchengröße im Bereich von 0,4 μm und 10 μm sowie
d.)3. optional weitere Füllstoffe, die nicht d.)1. und d.)2. entsprechen.

6. Verwendung einer radikalisch härtbaren Zusammensetzung nach Anspruch 5, wobei Komponente d.)1. zumindest teilweise nicht agglomeriert und/oder nicht aggregiert ist.

7. Verwendung einer radikalisch härtbaren Zusammensetzung nach einem der Ansprüche 5 oder 6, wobei Komponente d.)2. zwei oder mehr Mikrofraktionen enthält, wobei eine oder mehrere erste Mikrofraktionen jeweils eine mittlere Partikelgröße im Bereich von 1 bis 10 μm, vorzugsweise 1 bis 5 μm und wobei eine oder mehrere zweite Mikropartikelfraktionen jeweils eine mittlere Partikelgröße im Bereich von größer 0,4 μm bis kleiner 1 μm, vorzugsweise 0,5 μm bis 0,8 μm besitzen.

8. Verwendung einer radikalisch härtbaren Zusammensetzung nach Anspruch 7, wobei das Verhältnis der Gesamtmasse der ersten Mikropartikelfraktion(en) zur Gesamtmasse der zweiten Mikropartikelfraktion(en) im Bereich von 1:1 bis 12:1, vorzugsweise im Bereich von 1,5:1 bis 8:1 liegt.

9. Verwendung einer radikalisch härtbaren Zusammensetzung nach einem der Ansprüche 7 oder 8, wobei das Verhältnis der mittleren Korngröße der oder einer ersten Mikropartikelfraktion zur mittleren Korngröße der oder einer zweiten Mikropartikelfraktion der Komponente d.)2. im Bereich von 1,5:1 bis 10:1, vorzugsweise im Bereich von 2:1 bis 5:1 liegt.

10. Verwendung einer radikalisch härtbaren Zusammensetzung nach einem der Ansprüche 5 bis 9, wobei zumindest ein Teil der Mikropartikel der Komponente d.)2. organisch oberflächenmodifizierte Partikel, vorzugsweise silanisierte Partikel sind und/oder zumindest ein Teil der Mikropartikel der Komponente d.)2. Dentalglaspartikel sind, wobei zumindest ein Teil der Mikropartikel der Komponente d.)2. organisch oberflächenmodifizierte Dentalglaspartikel, vorzugsweise silanisierte Dentalglaspartikel sind.

11. Verwendung einer radikalisch härtbaren Zusammensetzung nach einem der Ansprüche 5 bis 10, wobei die Zusammensetzung die Komponente

d.)1. zu 1 - 50 Gew.-%, vorzugsweise 2 - 40 Gew.-%
d.)2. zu 10 - 84 Gew.-%, vorzugsweise 20 - 65 Gew.-%
d.)3. zu 0 - 30 Gew.-%, vorzugsweise 0 - 15 Gew.-%

enthält und wobei die Gewichtsprozentangaben auf die Gesamtmasse der radikalisch härtbaren dentalen Zusammensetzung bezogen sind.

12. Verwendung einer radikalisch härtbaren Zusammensetzung nach einem der vorangehenden Ansprüche, **dadurch gekennzeichnet, dass** die Komponente d.) röntgenopake Anteile enthält.

13. Verwendung einer radikalisch härtbaren Zusammensetzung nach Anspruch 12, wobei die röntgenopaken Anteile nanoskaliges $YbF_3$ und/oder $BaSO_4$ sind.

14. Verwendung einer radikalisch härtbaren Zusammensetzung nach einem der vorangehenden Ansprüche, wobei die radikalisch polymerisierbaren Gruppen Acrylat- oder Methacrylatgruppen sind.

15. Verwendung einer radikalisch härtbaren Zusammensetzung nach einem der Ansprüche 1 bis 14, wobei das generative Fertigungsverfahren die Stereolithographie, das digital light processing, die Polyjettechnologie, die Galvanometer type scanning method, die Mikrostereolithographie, das Multi-Jet Modelling, das selektive Lasersintern, das 3D-Printing, das Fused Deposition Modelling, das 3D-Plotting, das Laminated Object Manufacturing oder das Film Transfer Imaging umfasst.

## Claims

**1.** The use of a free-radically curable composition comprising

a.) chain-like and/or cyclic and/or cage-type polysiloxanes substituted by free-radically polymerizable groups and having at least 3 silicon atoms and/or mixed forms thereof, wherein the polysiloxanes a.) are obtained by hydrolysis or partial hydrolysis and subsequent condensation or co-condensation of one, two, three or more compounds $R^1_aR^2_bSiX_c$ with
X: halogen or alkoxy,
$R^2$: alkyl, alkenyl, aryl, alkylaryl, arylalkyl, wherein different $R^2$ may be the same or different,
$R^1$: YZ,
Z: free-radically polymerizable group selected from the structural elements $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ and $-NH-(C=O)-C(CH_3)=CH_2$,
wherein different Z may be the same or different,

a: 1 or 2,
b: 0 or 1,
c: 2 or 3,

$$a + b + c = 4,$$

Y: a connecting element which links the silicon atom to the free-radically polymerizable group and consists of an alkylene group,
wherein the alkylene group is an unsubstituted, linear, straight-chain or branched hydrocarbyl chain or
wherein the alkylene group is an unsubstituted hydrocarbyl group interrupted by a urethane group, urea group, ester group, thiourethane group or amide group or
wherein the alkylene group is a hydrocarbyl group substituted by a hydroxyl group or this hydroxyl group has been esterified or etherified or
wherein the alkylene group is a hydrocarbyl group interrupted by an oxygen atom and/or nitrogen atom and/or sulfur atom and/or ester groups and/or thioester groups and substituted by a hydroxyl group or this hydroxyl group has been esterified or etherified and
wherein different Y may be the same or different and
wherein Y contains 20 or fewer carbon atoms and wherein YZ is chosen such that Z always has a maximum number of atoms.
b.) disiloxanes substituted by free-radically polymerizable groups and having the following structure:

$$R^1_aR^2_{(3-a)}Si-O-SiR^2_{(3-b)}R^1_b$$

with

$R^2$: alkyl, alkenyl, aryl, alkylaryl, arylalkyl, wherein different $R^2$ may be the same or different,
$R^1$: YZ,
Z: free-radically polymerizable group selected from the structural elements $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ and $-NH-(C=O)-C(CH_3)=CH_2$,
wherein different Z may be the same or different,

a: 1 or 2,
b: 1 or 2,

Y: a connecting element which links the silicon atom to the free-radically polymerizable group and consists of an alkylene group,
wherein the alkylene group is an unsubstituted, linear, straight-chain or branched hydrocarbyl chain or
wherein the alkylene group is an unsubstituted hydrocarbyl group interrupted by a urethane group, urea group, ester group, thiourethane group or amide group or
wherein the alkylene group is a hydrocarbyl group substituted by a hydroxyl group or this hydroxyl group

has been esterified or etherified or
wherein the alkylene group is a hydrocarbyl group interrupted by an oxygen atom and/or nitrogen atom and/or sulfur atom and/or ester groups and/or thioester groups and substituted by a hydroxyl group or this hydroxyl group has been esterified or etherified and
wherein different Y may be the same or different and
wherein Y contains 20 or fewer carbon atoms and
wherein YZ is chosen such that Z always has a maximum number of atoms,

c.) optionally one, two, three or more free-radically curable monomers having no silicon atom,
d.) 85 percent by weight or less of fillers based on the total weight of the free-radically curable dental composition,
e.) initiators and/or catalysts for the free-radical polymerization and
f.) further customary additives

in an additive manufacturing method for production of a dental molding, wherein the dental molding is an inlay, an onlay, a veneer, a crown, a bridge, an artificial tooth, a denture, a scaffold, a temporary prosthesis, a partial or full prosthesis or else an orthodontic product.

**2.** The use of a free-radically curable composition according to claim 1, wherein the composition contains:

constituent a.) in an amount of 10%-70% by weight, preferably 10%-35% by weight,
constituent b.) in an amount of 2%-40% by weight, preferably 2%-25% by weight,
constituent c.) in an amount of 0%-30% by weight, preferably 0%-20% by weight,
constituent d.) in an amount of 0%-85% by weight, preferably 40%-78% by weight,
constituent e.) in an amount of 0.001%-5% by weight, preferably 0.1%-2% by weight and
constituent f.) in an amount of 0.001%-20% by weight, preferably 0.001%-10% by weight,

and wherein the respective percentages by weight are based on the total mass of the composition.

**3.** The use of a free-radically curable composition according to claim 1 or 2, wherein the composition contains, as constituent b.), 1,3-bis(3-methacryloyloxypropyl)tetramethyldisiloxane.

**4.** The use of a free-radically curable composition according to claim 1, 2 or 3, wherein the composition does not contain any constituent c.).

**5.** The use of a free-radically curable composition according to any of the preceding claims, containing a mixture of fillers d. comprising:

d.)1. organically surface-modified inorganic nanoparticles having an average particle size of less than 200 nm,
d.)2. inorganic microparticles having an average particle size in the range from 0.4 μm to 10 μm, and
d.)3. optionally further fillers which do not correspond to d.)1. and d.)2.

**6.** The use of a free-radically curable composition according to claim 5, wherein component d.)1. is at least partly in nonagglomerated and/or nonaggregated form.

**7.** The use of a free-radically curable composition according to claim 5 or 6, wherein component d.)2. contains two or more micro fractions, wherein a first micro fraction or each of a plurality of first micro fractions has an average particle size in the range from 1 to 10 μm, preferably 1 to 5 μm, and wherein a second microparticle fraction or each of a plurality of second microparticle fractions has an average particle size in the range from greater than 0.4 μm to less than 1 μm, preferably 0.5 μm to 0.8 μm.

**8.** The use of a free-radically curable composition according to claim 7, wherein the ratio of the total mass of the first microparticle fraction(s) to the total mass of the second microparticle fraction(s) is in the range from 1:1 to 12:1, preferably in the range from 1.5:1 to 8:1.

**9.** The use of a free-radically curable composition according to claim 7 or 8, wherein the ratio of the average particle size of the first or a first microparticle fraction to the average particle size of the second or a second microparticle fraction of component d.)2. is in the range from 1.5:1 to 10:1, preferably in the range from 2:1 to 5:1.

**10.** The use of a free-radically curable composition according to any of claims 5 to 9, wherein at least some of the microparticles of component d.)2. are organically surface-modified particles, preferably silanized particles, and/or at least some of the microparticles of component d.)2. are dental glass particles, wherein at least some of the microparticles of component d.)2. are organically surface-modified dental glass particles, preferably silanized dental glass particles.

**11.** The use of a free-radically curable composition according to any of claims 5 to 10, wherein the composition contains:

constituent d.)1. in an amount of 1%-50% by weight, preferably 2%-40% by weight,
constituent d.)2. in an amount 10%-84% by weight, preferably 20%-65% by weight,
constituent d.)3. in an amount 0%-30% by weight, preferably 0%-15% by weight,

and wherein the percentages by weight are based on the total mass of the free-radically curable dental composition.

**12.** The use of a free-radically curable composition according to any of the preceding claims, wherein component d.) contains x-ray-opaque fractions.

**13.** The use of a free-radically curable composition according to claim 12, wherein the x-ray-opaque fractions are nanoscale $YbF_3$ and/or $BaSO_4$.

**14.** The use of a free-radically curable composition according to any of the preceding claims, wherein the free-radically polymerizable groups are acrylate or methacrylate groups.

**15.** The use of a free-radically curable composition according to any of claims 1 to 14, wherein the additive manufacturing method is stereolithography, digital light processing, polyjet technology, the galvanometer-type scanning method, micro-stereolithography, multijet modeling, selective laser sintering, 3D printing, fused deposition modeling, 3D plotting, laminated object manufacturing or film transfer imaging.

## Revendications

**1.** Utilisation d'une composition durcissable par voie radicalaire, contenant :

a.) des polysiloxanes linéaires et/ou cycliques et/ou sous la forme de cage substitués avec des groupes polymérisables par voie radicalaire, contenant au moins 3 atomes de silicium et/ou leurs formes mixtes, les polysiloxanes a.) étant obtenus par hydrolyse ou hydrolyse partielle, puis condensation ou co-condensation d'un, de deux, de trois ou de davantage de composés $R^1_aR^2_bSiX_c$, avec
X : halogène ou alcoxy,
$R^2$ : alkyle, alcényle, aryle, alkylaryle, arylalkyle, différents $R^2$ pouvant être identiques ou différents,
$R^1$ : YZ,
Z : groupe polymérisable par voie radicalaire, choisi parmi les éléments structuraux $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ et $-NH-(C=O)-C(CH_3)=CH_2$, différents Z pouvant être identiques ou différents,

a : 1 ou 2,
b : 0 ou 1,
c : 2 ou 3,

$$a+b+c = 4,$$

Y : un élément de liaison, qui relie l'atome de silicium avec le groupe polymérisable par voie radicalaire et est constitué par un groupe alkylène,
le groupe alkylène étant une chaîne hydrocarbonée non substituée, continue, linéaire ou ramifiée, ou
le groupe alkylène étant un groupe hydrocarboné non substitué, interrompu par un groupe uréthane, un groupe urée, un groupe ester, un groupe thiouréthane ou un groupe amide, ou
le groupe alkylène étant un groupe hydrocarboné substitué par un groupe hydroxyle, ou ce groupe hydroxyle étant estérifié ou éthérifié, ou

le groupe alkylène étant un groupe hydrocarboné interrompu par un atome d'oxygène et/ou un atome d'azote et/ou un atome de soufre et/ou des groupes esters et/ou des groupes thioester et substitué par un groupe hydroxyle, ou ce groupe hydroxyle étant estérifié ou éthérifié, et
différents Y pouvant être identiques ou différents, et
Y contenant 20 atomes de carbone ou moins, et
YZ étant choisi de telle sorte que Z comprenne toujours autant d'atomes que possible,
b.) des disiloxanes substitués avec des groupes polymérisables par voie radicalaire de la structure suivante :

$$R^1_aR^2_{(3-a)}Si\text{-}O\text{-}SiR^2_{(3-b)}R^1_b$$

avec

$R^2$ : alkyle, alcényle, aryle, alkylaryle, arylalkyle, différents $R^2$ pouvant être identiques ou différents,
$R^1$ : YZ,
Z : groupe polymérisable par voie radicalaire, choisi parmi les éléments structuraux $-O-(C=O)-CH=CH_2$, $-O-(C=O)-C(CH_3)=CH_2$, $-(C=O)-CH=CH_2$, $-(C=O)-C(CH_3)=CH_2$,$-CH=CH_2$, $-C(CH_3)=CH_2$, $-NH-(C=O)-CH=CH_2$ et $-NH-(C=O)-C(CH_3)=CH_2$, différents Z pouvant être identiques ou différents,

a : 1 ou 2,
b : 1 ou 2,

Y : un élément de liaison, qui relie l'atome de silicium avec le groupe polymérisable par voie radicalaire et est constitué par un groupe alkylène,
le groupe alkylène étant une chaîne hydrocarbonée non substituée, continue, linéaire ou ramifiée, ou
le groupe alkylène étant un groupe hydrocarboné non substitué, interrompu par un groupe uréthane, un groupe urée, un groupe ester, un groupe thiouréthane ou un groupe amide, ou
le groupe alkylène étant un groupe hydrocarboné substitué par un groupe hydroxyle, ou ce groupe hydroxyle étant estérifié ou éthérifié, ou
le groupe alkylène étant un groupe hydrocarboné interrompu par un atome d'oxygène et/ou un atome d'azote et/ou un atome de soufre et/ou des groupes esters et/ou des groupes thioester et substitué par un groupe hydroxyle, ou ce groupe hydroxyle étant estérifié ou éthérifié, et
différents Y pouvant être identiques ou différents, et
Y contenant 20 atomes de carbone ou moins, et
YZ étant choisi de telle sorte que Z comprenne toujours autant d'atomes que possible,

c.) éventuellement un, deux, trois ou davantage de monomères durcissables par voie radicalaire sans atome de silicium,
d.) 85 pour cent en poids ou moins de charges, comprenant, par rapport au poids total de la composition dentaire durcissable par voie radicalaire,
e.) des initiateurs et/ou des catalyseurs pour la polymérisation radicalaire, et
f.) d'autres additifs usuels,

dans un procédé de production génératif pour la fabrication d'une pièce moulée dentaire, la pièce moulée dentaire étant un inlay, un onlay, une facette, une couronne, un bridge, une dent artificielle, un élément préfabriqué de dent, une structure, un élément provisoire, une prothèse partielle ou totale, ainsi qu'un produit KFO.

**2.** Utilisation d'une composition durcissable par voie radicalaire selon la revendication 1, dans laquelle la composition contient le composant :

a.) à hauteur de 10 à 70 % en poids, de préférence 10 à 35 % en poids,
b.) à hauteur de 2 à 40 % en poids, de préférence 2 à 25 % en poids,
c.) à hauteur de 0 à 30 % en poids, de préférence 0 à 20 % en poids,
d.) à hauteur de 0 à 85 % en poids, de préférence 40 à 78 % en poids,
e.) à hauteur de 0,001 à 5 % en poids, de préférence 0,1 à 2 % en poids, et
f.) à hauteur de 0,001 à 20 % en poids, de préférence 0,001 à 10 % en poids,

les pourcentages en poids respectifs se rapportant à la masse totale de la composition.

**3.** Utilisation d'une composition durcissable par voie radicalaire selon l'une quelconque des revendications 1 ou 2, dans laquelle la composition contient en tant que constituant b.) le 1,3-bis(3-méthacryloxypropyl)tétraméthyldisiloxane.

**4.** Utilisation d'une composition durcissable par voie radicalaire selon l'une quelconque des revendications 1, 2 ou 3, dans laquelle la composition ne contient pas de constituant c.).

**5.** Utilisation d'une composition durcissable par voie radicalaire selon l'une quelconque des revendications précédentes, contenant un mélange de charges d. comprenant :

d.)1. des nanoparticules inorganiques modifiées organiquement en surface ayant une taille de particule moyenne inférieure à 200 nm,
d.)2. des microparticules inorganiques ayant une taille de particule moyenne dans la plage allant de 0,4 μm à 10 μm, et
d.)3. éventuellement d'autres charges, qui ne correspondent ni à d.)1., ni à d.)2.

**6.** Utilisation d'une composition durcissable par voie radicalaire selon la revendication 5, dans laquelle le composant d.)1. est au moins partiellement non aggloméré et/ou non agrégé.

**7.** Utilisation d'une composition durcissable par voie radicalaire selon l'une quelconque des revendications 5 ou 6, dans laquelle le composant d.)2. contient deux microfractions ou plus, une ou plusieurs premières microfractions présentant chacune une taille de particule moyenne dans la plage allant de 1 à 10 μm, de préférence de 1 à 5 μm, et une ou plusieurs deuxièmes fractions de microparticules présentant chacune une taille de particule moyenne dans la plage allant de plus de 0,4 μm à moins de 1 μm, de préférence de 0,5 μm à 0,8 μm.

**8.** Utilisation d'une composition durcissable par voie radicalaire selon la revendication 7, dans laquelle le rapport entre la masse totale de la ou des premières fractions de microparticules et la masse totale de la ou des deuxièmes fractions de microparticules se situe dans la plage allant de 1:1 à 12:1, de préférence dans la plage allant de 1,5:1 à 8:1.

**9.** Utilisation d'une composition durcissable par voie radicalaire selon l'une quelconque des revendications 7 ou 8, dans laquelle le rapport entre la taille de particule moyenne de la ou d'une première fraction de microparticules et la taille de particule moyenne de la ou d'une deuxième fraction de microparticules du composant d.)2. se situe dans la plage allant de 1,5:1 à 10:1, de préférence dans la plage allant de 2:1 à 5:1.

**10.** Utilisation d'une composition durcissable par voie radicalaire selon l'une quelconque des revendications 5 à 9, dans laquelle au moins une partie des microparticules du composant d.)2. sont des particules modifiées organiquement en surface, de préférence des particules silanisées, et/ou au moins une partie des microparticules du composant d.)2. sont des particules de verre dentaire, au moins une partie des microparticules du composant d.)2. étant des particules de verre dentaire modifiées organiquement en surface, de préférence des particules de verre dentaire silanisées.

**11.** Utilisation d'une composition durcissable par voie radicalaire selon l'une quelconque des revendications 5 à 10, dans laquelle la composition contient le composant :

d.)1. à hauteur de 1 à 50 % en poids, de préférence 2 à 40 % en poids,
d.2.) à hauteur de 10 à 84 % en poids, de préférence 20 à 65 % en poids,
d.3.) à hauteur de 0 à 30 % en poids, de préférence 0 à 15 % en poids,

les données de pourcentage en poids se rapportant à la masse totale de la composition dentaire durcissable par voie radicalaire.

**12.** Utilisation d'une composition durcissable par voie radicalaire selon l'une quelconque des revendications précédentes, **caractérisée en ce que** le composant d.) contient des fractions opaques aux rayons X.

**13.** Utilisation d'une composition durcissable par voie radicalaire selon la revendication 12, dans laquelle les fractions opaques aux rayons X sont du $YbF_3$ et/ou $BaSO_4$ nanométrique.

**14.** Utilisation d'une composition durcissable par voie radicalaire selon l'une quelconque des revendications précédentes, dans laquelle les groupes polymérisables par voie radicalaire sont des groupes acrylate ou méthacrylate.

15. Utilisation d'une composition durcissable par voie radicalaire selon l'une quelconque des revendications 1 à 14, dans laquelle le procédé de production génératif comprend la stéréolithographie, le Digital Light Processing, la technologie polyjet, le procédé de numérisation de type galvanomètre, la microstéréolithographie, la modélisation multi-jet, le frittage laser sélectif, l'impression 3D, le dépôt de fils (FDM), le tracé 3D, le Laminated Object Manufacturing ou le Film Transfer Imaging.

Abbildung 1: E-Modul der Beispiele 1-4

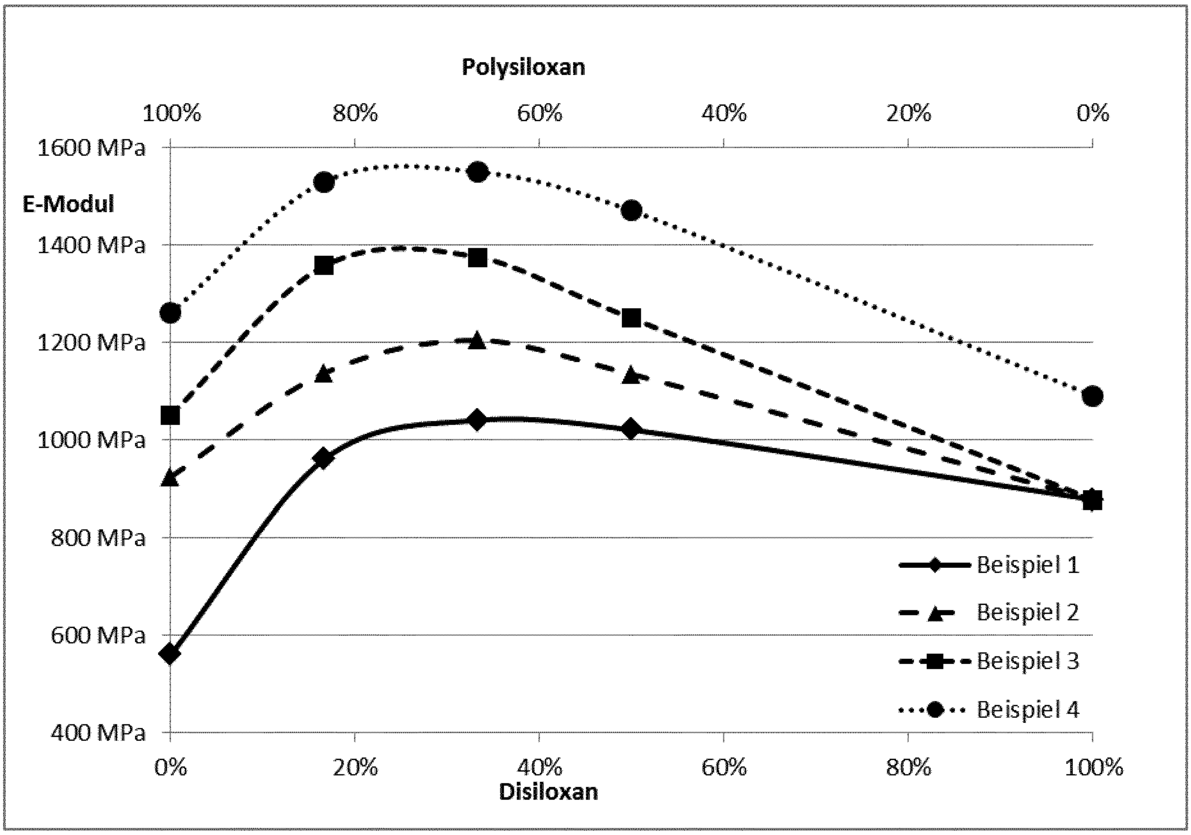

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4575330 A
- DE 69704623 T2
- WO 2013153183 A2
- DE 19938463 A1
- DE 19950284 A1
- DE 4133494 C2
- DE 3903407 A1
- DE 10114290 B4
- DE 102012012346 A1
- DE 19934407 A1
- DE 2758414 A1
- DE 4416857 C1
- DE 19860364 C2
- EP 1874847 B1
- EP 1685182 B1
- WO 2013041723 A1
- WO 2013053693 A1
- DE 102014210432
- DE 3941629 A1
- EP 11183333 A
- EP 11183328 A
- EP 11183345 A
- EP 11183338 A
- EP 11183342 A
- EP 11188086 A
- WO 2005011621 A1
- DE 102006019092 A1
- DE 3941629 C2
- DE 102006019092
- DE 60116142
- DE 3801511 C2
- DE 102006050153 A1
- EP 0184095 B1
- DE 4231579 C2
- EP 0366977 B1
- US 7081485 B2
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- EP 1905415 A
- US 4772530 A
- US 4954414 A
- US 4874450 A
- US 5055372 A
- US 5057393 A
- EP 0783880 B1
- DE 10119831 A1
- EP 1563821 A1


### In der Beschreibung aufgeführte Nicht-Patentliteratur


- **A. GEBHARD.** Generative Fertigungsverfahren. Carl Hanser Verlag, 2013
- **J.M. ANTONUCI ; J.W. STANSBURY ; S. VENZ.** Synthesis and properties of a polyfluorinated prepolymer multifunctional urethan methacrylate. *Polymeric Materials Science and Engineering,* 1988, vol. 59, 388-396
- **J.S. KUO ; J.M. ANTONUCCI ; W. WU.** Evaluation of siloxane containing dental composites. *Journal of Dental Research Abstracts,* 1985
- **X. LIANG ; F. LIU ; J. HE.** Synthesis of none Bisphenol A structure dimethacrylate monomer and characterization for dental composite applications. *Dental Materials,* 2014, vol. 30, 917-925
- **DEBORAH HUCK-JONES ; RENATE HESSEMANN.** Chemische Identität einzelner Partikel. *Nachrichten aus der Chemie,* September 2014, vol. 62, 886, , 887
- **J.-P. FOUASSIER.** Photoinitiation, Photopolymerization and Photocuring. Hanser Publishers, 1995
- Radiation Curing in Polymer Science and Technology. Elsevier Applied Science, 1993, vol. II
- **WATTS DC ; CASH AJ.** Determination of polymerization kinetics in visible-light cured materials: Methods development. *Dent Mater,* 1991, vol. 7, 281-287